# EUROPEAN PATENT APPLICATION

(11) **EP 1 394 176 A1**
(43) Date of publication of application: **03.03.2004**
(21) Application number: 02019243.1
(22) Date of filing: 27.08.2002
(51) Int. Cl.: C07K 14/47

(54) **Novel mucin binding polypeptides derived from Lactobacillus johnsonii**

(71) Applicant: NESTEC S.A., 1800 Vevey (CH)
(72) Inventor: Granato, Dominique-Anne, 1091 Grandvaux (CH)
(74) Representative: Becker Kurig Straus

(57) **Abstract**

The present invention relates to novel genes and polypeptides, including elongation factor efTu, heat shock protease clpE and pyruvate kinase like proteins of Lactobacillus johnsonii involved in binding of the bacteria to mucins and to the corresponding genes. In particular, the invention pertains to the use of the present genes in the preparation of polypeptides having the capacity to bind to mucins.

## Description

The present invention relates to novel genes of Lactobacillus johnsonii encoding polypeptides involved in binding of the bacteria to mucins. These polypeptides belong to the protein families of elongation factors, heat shock protease clpE, or pyruvate kinases. In particular, the invention pertains to the use of the present genes in the preparation of polypeptides having the capacity to bind to mucins.

Due to their widespread employment in the preparation and preservation of food products, lactic acid bacteria have become a focus of intensive investigation. In nature, these bacteria are usually found in milk of mammals and plants, but are also prevalent in the intestine of human beings, where they represent a major constituent of the micro-flora.

In the recent past some particular strains of lactic acid bacteria have been found to exhibit favorable properties to man and animals upon ingestion. In particular, specific strains of the genus Lactobacillus or Bifidobacterium have been established to endure conditions prevailing in the different areas of the gastro-intestinal tract in a viable and live form and to be able to adhere to and colonize it. These kinds of strains are generically referred to as probiotics.

The gastro-intestinal tract is generally designed to function as a barrier against pathogens or exogenous antigens derived from microorganisms or food material and comprises a micro-flora, i.e. a complex ecosystem consisting of hundreds of bacterial species. Moreover, the gastro-intestinal tract and the micro-flora, respectively, is involved in the generation of immuno-physiologic reactions, such as eliciting immune responses or being implicated in the pathogenesis of several chronic conditions, such as inflammatory bowel disease.

This knowledge has led to the introduction of novel therapeutic interventions based on modifying the colonic flora by consumption of probiotic microorganisms in order to improve the colonic performance and to prevent and treat intestinal disorders.

So far, a variety of reports have been issued, showing that specific probiotic microbes may alleviate or prevent diverse intestinal disorders and reduce the risk of some intestinal diseases. Some among the most recent publications on *in vivo studies* with probiotics describe their beneficial effect on the immune defenses (Majamaa et al., J. Allergy Clin Immunol 1997 Feb;99(2):179-85); Schiffrin et al., Am J Clin Nutr 1997 Aug;66(2):515S-520S), on allergy (Majamaa et al., supra; Kalliomaki et al., Lancet 2001 Apr 7;357(9262):1076-9) or on the proliferation of intestinal cells (Ichikawa et al., Dig Dis Sci 1999 Oct;44(10):2119-23). Moreover, said probiotics have been determined to have a role in pathogen exclusion (Mangiante et al, Chir Ital. 1999 May-Jun;51(3):221-6), and in lipid metabolism (Taranto et al., Dig Dis Sci 2000;45(8):1617-22).

In EP 0 768 375, a specific strain of the genus Bifidobacterium is disclosed, which is reported to become implanted in the intestinal flora and to assist in a modulation of the host's immune system, being capable to competitively exclude adhesion of pathogenic bacteria to intestinal cells, thus supporting the maintenance of the individual's health.

In addition, in EP 0 577 903 the use of such a lactic acid bacteria having the ability of replacing Helicobacter pylori, an approved origin for the development of ulcer, for the therapeutic or prophylactic treatment of ulcer associated with the action of Helicobacter pylori is disclosed.

The precise mechanisms underlying the beneficial effects of probiotics on their hosts have not yet been elucidated. It is assumed that a probiotic effect can best be achieved if the organisms adhere to mucosal cells and at least transiently stay in the gastro-intestinal tract. However, at present there is no evidence that exogenously administered probiotics are really capable to adhere to the mucosa or mucosal cells *in vivo*. Instead, they might pass into the feces without having adhered or multiplied (Bezkorovainy et al., Am J Clin. Nutr. 2001;73(2 Suppl):399S-405S).

In general, in view of the known valuable properties of probiotic strains obtaining a more detailed information relating to the biology of these strains is desired, in particular as regards the interaction with the hosts, that is the capability to adhere to the intestine's mucosa, which information will allow a better understanding of these mechanisms.

Consequently, a problem of the present invention is to provide a means with which probiotic strains attach to intestinal structures.

This problem has been solved by providing several novel genes of Lactobacillus johnsonii all of which encode surface proteins, i.e. membrane anchored or other polypeptides, allowing the micro-organism to bind and adhere to mucins.

Mucins are large glycoprotein molecules having a molecular weight of more than about 1,000,000, being present on the surface of a number of epithelial cells, including those of the gastrointestinal tract, the lung or the uterine cervicex. Their main biological function resides in protecting and lubricating the epithelial cells thereunder, inhibiting bacteria from infestation of the tissue and rendering waterproof capability to the mucosa. The polypeptide portion of mucins contains threonine, serine and proline as a major constituent and often exhibits a tandemly repeated unit of amino acids. The oligosaccharide side chains, which comprise up to 80% of the molecular weight of the mucin glycoproteins, consist of hexose, fucose, hexosamine, sialic acid and sulphate ester and show a high variability. In humans at least four mucin families have been found so far, known as MUC1, MUC2, MUC3 and MUC4.

In the figures,
Fig. 1 shows a map of the plasmid pDP649 constructed for the over-expression of the L. johnsonii CNCM I-1225 elongation factor Tu with the carboxyl-terminal six-times His tag;
Figs. 2A and 2B show the detection of CNCM I-1225 cell wall proteins bound to differentiated Caco-2 and HT29 cells. Differentiated Caco-2 cells (A) or HT29 cells (B) were incubated with buffer alone (a and c) or with a CNCM I-1225 cell wall extract from CNCM I-1225 (b and d) at pH 7.2 (a and b) or at pH 5.0 (c and d). Bound proteins were eluted as described, submitted to SDS-PAGE and analyzed by Western blot using anti-CNCM I-1225 surface antibodies.

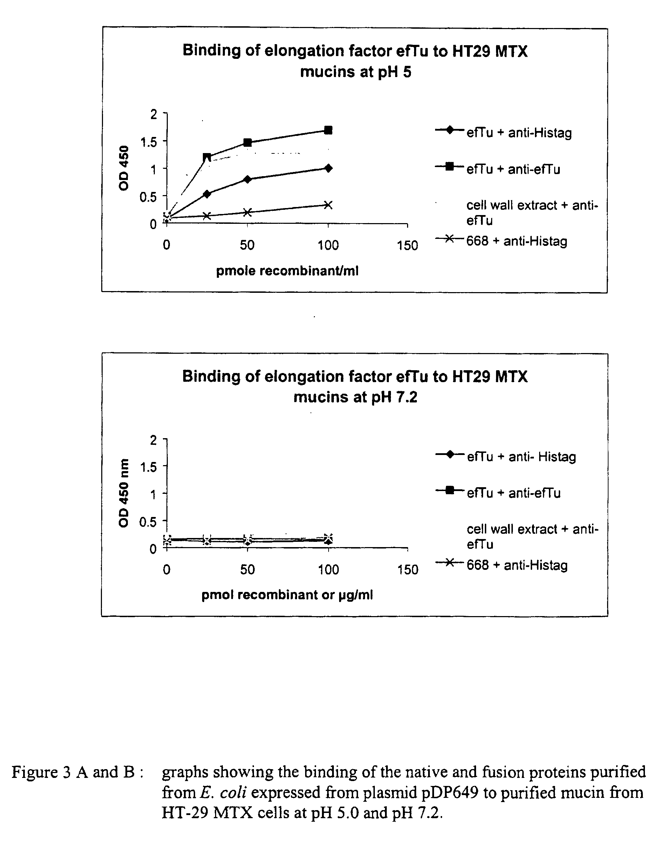

Figs. 3 A and B show graphs showing the binding of the native and fusion proteins purified from E. coli expressed from plasmid pDP649 to purified mucin from HT-29 MTX cells at pH 5.0 and pH 7.2.
Figs. 4 A and B show graphs showing the binding of the native and fusion proteins purified from E. coli expressed from plasmid pDP649 to HT29 cells at pH 5.0 and pH 7.2.
Figs. 5A and 5 B show graphs showing the binding of the native and fusion proteins purified from E. coli expressed from plasmid pDP649 to Caco-2 cells at pH 5.0 and pH 7.2.

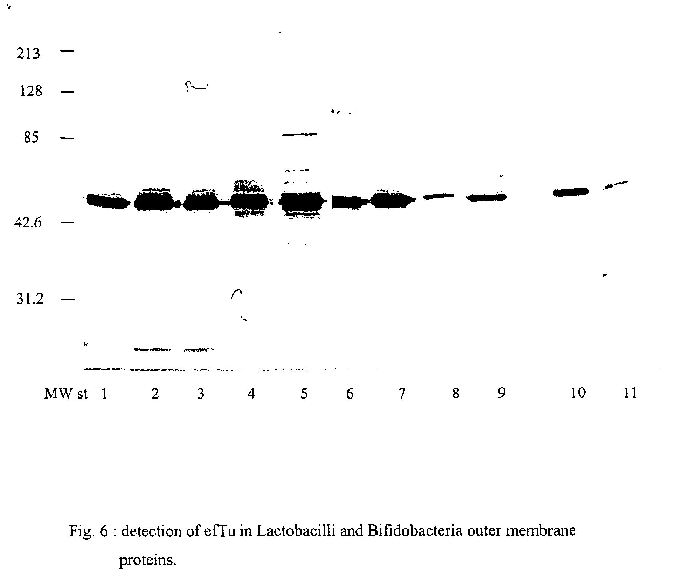

Fig. 6 shows the detection of efTu in Lactobacilli and Bifidobacteria outer membrane proteins. 1 µg of guanidine-HCl extract of outer membrane proteins of lactobacilli and Bifidobacteria was run on 10% SDS-PAGE, and analysed by Western blot using anti-efTu antiserum.1: *L. johnsonii* CNCM I-1225, 2 *L johnsonii* 1657, 3: *L. johnsonii* 1680, 4: *L. acidophilus* CNCM I-2332, 5: *L. acidophilus* 12, 6: *L reuteri* 2458, 7: *L. gasseri* 907, 8: *L. paracasei* CNCM I-2116, 9: *L. helveticus* 1643, 10: Bifidobacteria 362, 11: Bifidobacteria CNCM I-2170.

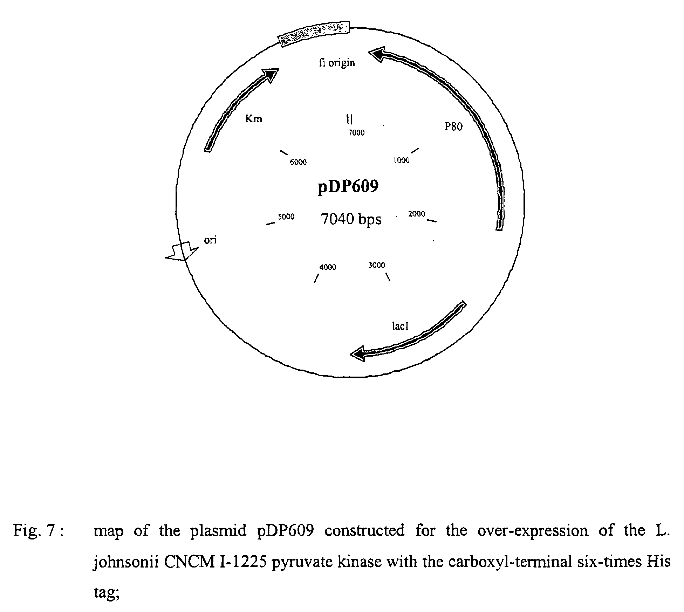

Fig. 7 shows a map of the plasmid pDP609 constructed for the over-expression of the L. johnsonii CNCM I-1225 pyruvate kinase with the carboxyl-terminal six-times His tag;

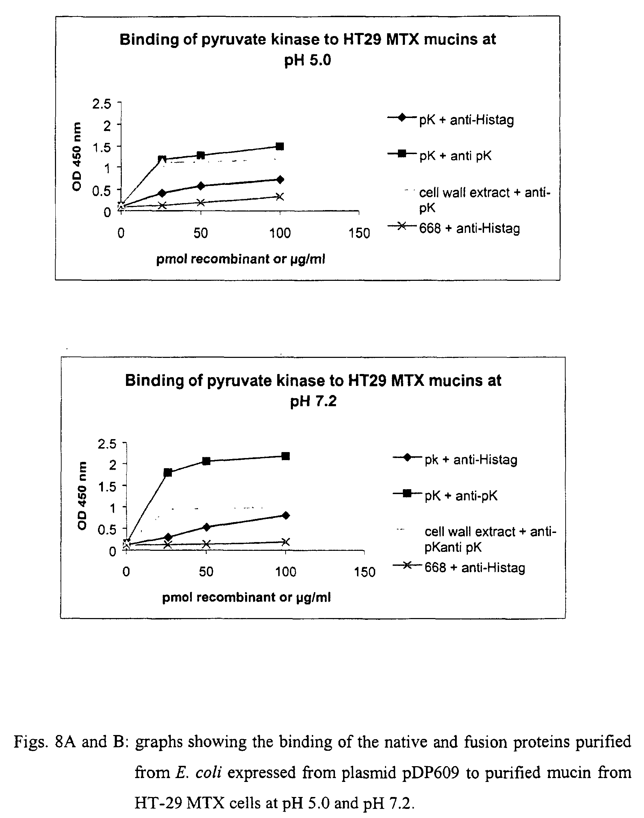

Figure 8 shows graphs showing the binding of the native and fusion proteins purified from E. coli expressed from plasmid pDP609 to purified mucin from HT-29 MTX cells at pH 5.0 and pH 7.2.

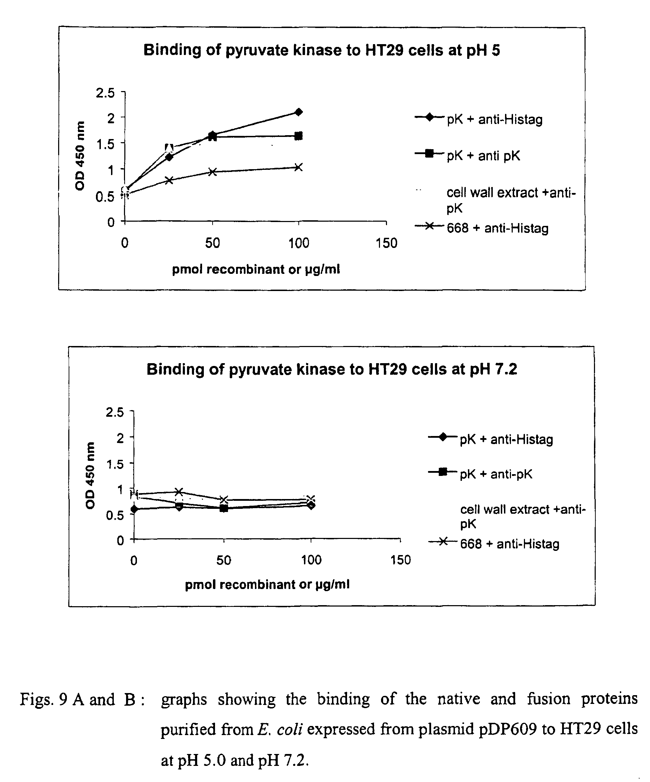

Figs. 9 A and 9 B show graphs showing the binding of the native and fusion proteins purified from *E. coli* expressed from plasmid pDP609 to HT29 cells at pH 5.0 and pH 7.2.

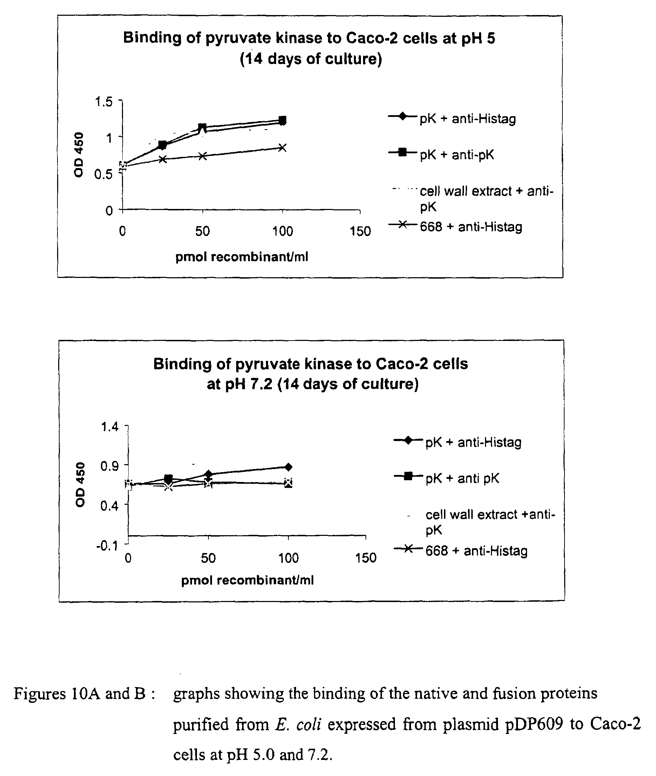

Figures 10A and 10 B show graphs showing the binding of the native and fusion proteins purified from E. coli expressed from plasmid pDP609 to Caco-2 cells at pH 5.0 and pH 7.2.

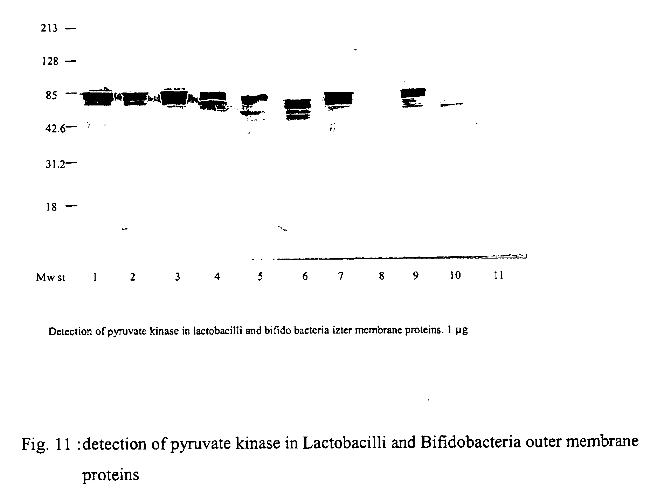

Fig. 11 shows the detection of pyruvate kinase in Lactobacilli and Bifidobacteria outer membrane proteins. 1 µg of guanidine-HCl extract of outer membrane proteins of Lactobacilli and Bifidobacteria was run on 10% SDS-PAGE, and analyzed by Western blot using anti-pyruvate kinase antiserum. 1: *L. johnsonii* CNCM I-1225, 2 *L johnsonii* 1657, 3: *L.* johnsonii 1680, 4: *L. acidophilus* CNCM-I 2332, 5: *L. acidophilus* 12, 6: *L reuteri* 2458, 7: *L. gasseri* 907, 8: *L. paracasei* CNCM-I-2116, 9: *L. helveticus* 1643, 10: Bifidobacteria 362, 11: Bifidobacteria CNCM-2170.

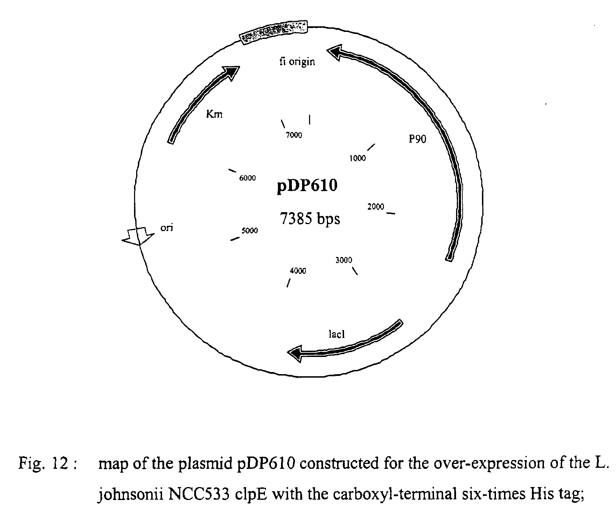

Fig. 12 shows a map of the plasmid pDP610 constructed for the over-expression of the L. johnsonii CNCM I-1225 clpE with the carboxyl-terminal six-times His tag;

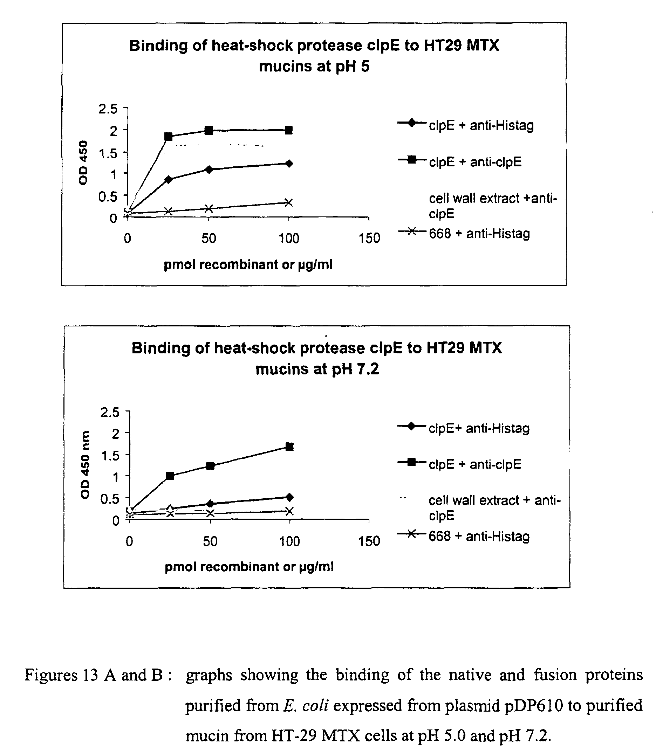

Figure 13 A and B shows graphs showing the binding of the native and fusion proteins purified from *E. coli* expressed from plasmid pDP610 to purified mucin from HT-29 MTX cells at pH 5.0 and pH 7.2.

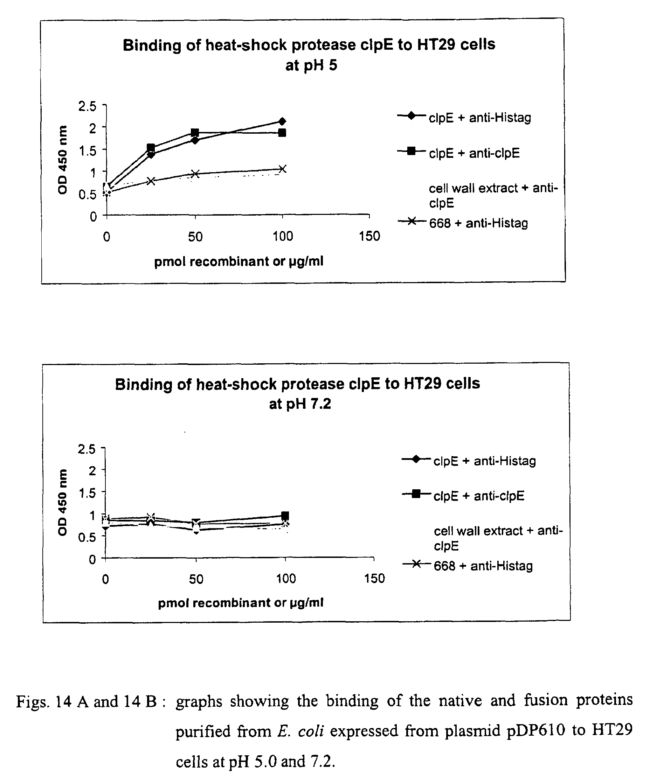

Figs. 14 A and 14 B show graphs showing the binding of the native and fusion proteins purified from *E. coli* expressed from plasmid pDP610 to HT29 cells at pH 5.0 and pH 7.2.

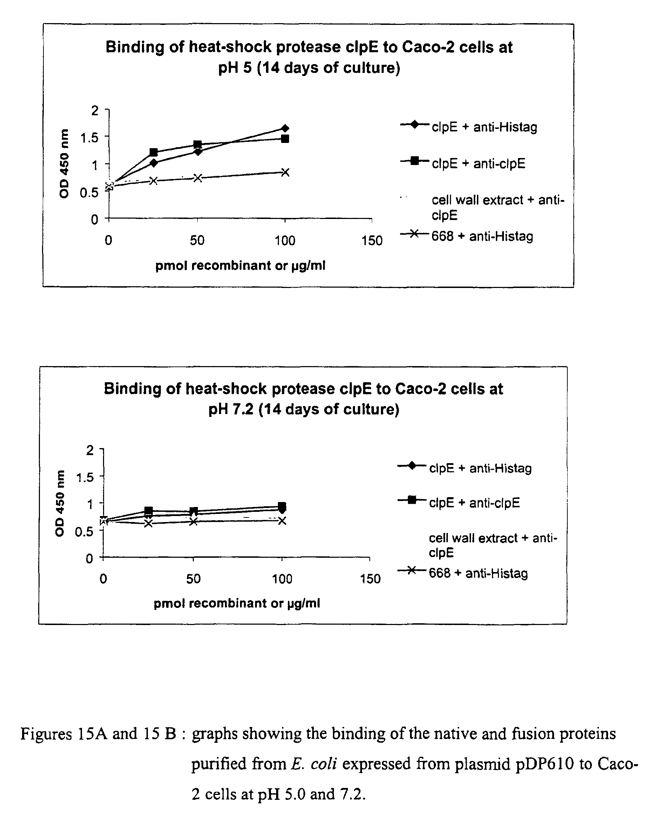

Figures 15A and 15 B show graphs showing the binding of the native and fusion proteins purified from *E. coli* expressed from plasmid pDP610 to Caco-2 cells at pH 5.0 and pH 7.2.

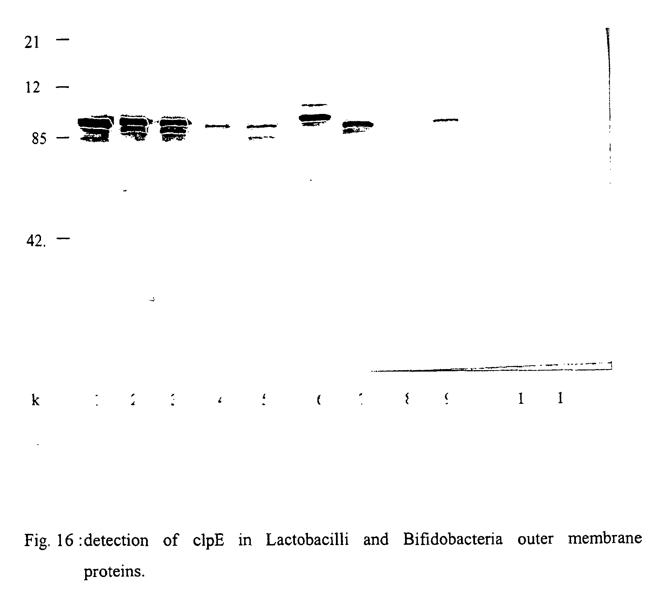

Fig. 16 shows the detection of clpE in Lactobacilli and Bifidobacteria outer membrane proteins. 1 µg of guanidine-HCl extract of outer membrane proteins of Lactobacilli and Bifidobacteria was run on 10% SDS-PAGE, and analysed by Western blot using anti-clpE antiserum.1: *L. johnsonii* CNCM I-1225, 2 *L johnsonii* 1657, 3: *L. johnsonii* 1680, 4: *L. acidophilus* CNCM-I 2332, 5: *L. acidophilus* 12, 6: *L reuteri* 2458, 7: *L. gasseri* 907, 8: *L. paracasei* CNCM-I-2116, 9: *L. helveticus* 1643, 10: Bifidobacteria 362, 11: Bifidobacteria CNCM-2170.

In the line of investigating the probiotic strain Lactobacillus johnsonii CNCM I-1225, the characterization of surface molecules has been focused upon, since some of said surface molecules were assumed to contribute to the probiotic properties of said strain. In this respect some membrane bound polypeptides have been identified, which upon peptide sequencing and association of the nucleotide sequence derived therefrom with the genomic sequence of the micro-organism showed an open reading frame that exhibits a moderate homology to a type of polypeptides utilized by micro-organisms as elongation factors (efTu) during transcription and translation. Surprisingly, this elongation factor type polypeptide was expressed not only in the cytosol, as typically expected, but also present on the surface of the probiotic strain investigated, which led to the notion that said elongation factor type polypeptides might have a novel, so far not recognized biological activity.

To identify the actual biological function of said new polypeptides in CNCM I-1225, the open reading frames of the corresponding genes were cloned into an expression vector, the polypeptides were recombinantly expressed in E.coli and collected. The resulting polypeptides were then subjected to a variety of different experimental conditions, such as e.g. binding assays to collagen or fibronectin, immune stimulation and pathogen exclusion.

Unexpectedly, an experiment, wherein the recombinant polypeptides were contacted with mucins, showed a substantial binding affinity so that the present gene products were deemed to be involved in allowing Lactobacillus johnsonii to adhere to the mucosal tissue in the intestinal tract.

Therefore, according to an embodiment the present invention provides a polynucleotide as identified by any of SEQ ID. Nos. 1 to 3 or variants thereof, which variants are homologous to the said sequences as shown in SEQ ID. Nos. 1 to 3 and will yield biological active polypeptides, that is polypeptides having the capacity to bind to mucins. According to most preferred embodiments the polynucleotides are identified by a sequence as identified by SEQ ID. No. 1 to 3.

In the context of the present application, the term "homology" shall designate a polynucleotide sequence if at least 90%, preferably at least 95 %, more preferably at least 97 %, even more preferably at least 98 %, of its nucleotide composition and nucleotide sequence corresponds or has identity to any of the sequences as identified by SEQ. ID. Nos. 1 to 3. Likewise, the term "variant" shall designate a polynucleotide having the above degree of homology to a polynucleotide as identified by any of the SEQ. ID. Nos. 1 to 3, or fragments thereof. The term "polynucleotide" as used in the present specification refers in general to polyribonucleotides and polydeoxyribonucleotides, and denotes an unmodified RNA or DNA or a modified RNA or DNA. In the context of the present invention, the term "fragment" is to be understood to refer to polynucleotides shorter than the polynucleotide as identified by SEQ ID Nos. 1 to 3.

The homology or sequence similarity or sequence identity of nucleotide sequences may easily be determined according to techniques well known in the art, e.g. by using established software or computer programs, e.g. the BestFit or Gap pairwise (GCG Wisconsin Package, Genetics Computer Group, 575 Science Drive, Madison, Wis. 53711). BestFit uses the local homology algorithm of Smith and Waterman, Advances in Applied Mathematics 2: 482-489 (1981), to find the best segment of identity or similarity between two sequences. Gap performs global alignments, all of one sequence with all of another similar sequence using the method of Needleman et al. (J. Mol. Biol. 48: (1970), 443-453). When using a sequence alignment program such as BestFit, to determine the degree of sequence homology, similarity or identity, the default setting may be used, or an appropriate scoring matrix may be selected to optimize identity, similarity or homology scores. Similarly, when using a program such as BestFit to determine sequence identity, similarity or homology between two different amino acid sequences, the default settings may be used, or an appropriate scoring matrix, such as blosum45 or blosum80, may be selected to optimize identity, similarity or homology scores.

Accordingly, the present invention also encompasses polynucleotides that hybridize, preferably under stringent conditions, to any of the nucleotide sequences as identified by SEQ ID. Nos. 1 to 3, or their complements. Hybridization procedures are well known in the art and instructions for identifying DNA sequences by means of hybridization may be found in the Handbook by Sambrook et al.: Molecular Cloning, A Laboratory Manual (Cold Spring Harbor Laboratory Press, 1989). The hybridization may take place under stringent conditions, that is to say only hybrids in which the probe and target sequence, i.e. the polynucleotides contacted with the probe, are at least 90% identical are formed. It is generally accepted that the stringency of the hybridization, including the washing steps, is influenced or determined by varying the buffer composition, the temperature, and the salt concentration. The hybridization reaction is preferably carried out under a relatively low stringency compared with the washing steps.

The polynucleotide sequences according to the invention may suitably be utilized as hybridization probes for RNA, cDNA and DNA, in order to isolate DNAs or genes from other species which exhibit a high degree of similarity to the sequence of the present gene. Moreover, oligonucleotides, derived from the present polynucleotide sequences do also represent suitable primers for performing a polymerase chain reaction (PCR) in order to synthesize DNA encoding a mucin binding polypeptides. Such oligonucleotides may contain of from 5 to 50 nucleotides, preferably of from 8 to 30, more preferably of from 10 to 20 nucleotides.

According to an embodiment, the present invention also provides vectors containing any of the polynucleotides as described herein and allowing propagation and amplification of the present polynucleotides. Suitable vectors are e.g. those, which are replicated in the micro-organisms, into which they have been introduced, at a high copy number. Also suitable are those vectors by means of which the process of gene amplification by integration in the chromosome can be employed. Preferably the vector shall also allow propagation in different micro-organisms (shuttle vector) and/or expression of the desired polypeptide. In order to enable a stable transmission to the progeny the vectors will also contain a selection marker, such as a gene conferring resistance to a specific agent, e.g. an antibiotic. Alternatively, a stable transmission may also be achieved in integrating the present polynucleotides into the host's chromosome. Preferably the vector will also allow expression of the polynucleotide contained therein, and includes regulatory regions, such as e.g. promotors or repressors, which allow transcription of the insert under specific conditions only. Thus, a desirable promotor may be inducible, so that the genetic material harboring the polynucleotide according to the present invention is amplified within the micro-organism, used for propagation and expression, while expression of the desired polypeptide is only effected, when the micro-organism has been cultivated to a certain extent. To this end a great number of polypeptides may be produced in the micro-organism, which might otherwise represent a burden of the growth thereof.

Examples of suitable vectors include e.g. pBR322, pUC, pNZ124, pGK12, pMG36, pSA3, pGKV110 and pGKV210.

According to another embodiment the present invention also pertains to a host cell containing a polynucleotide or a vector according to the present invention. In the context of the present invention a "host cell" as claimed shall designate a cell, that contains a polynucleotide or a vector as described herein, which polynucleotide or vector, respectively has not been originally endogenous. That is, the present invention embraces any kind of cell, into which a polynucleotide or vector according to the present invention has been introduced, which cell did not originally contain the present polynucleotides or vectors. Likewise, a host cell according to the present invention also includes a micro-organism, from which the present polynucleotide has been derived, but which now contains additional copies thereof.

Suitable host cells for use in the present invention comprise any kind of cell, in which the present polynucleotide may conveniently be propagated and/or expressed. Examples of such host cells are e.g. Lactococcus lactis, Lactobacillus delbruekii subsp. lactis and bulgaricus, Streptococcus thermophilus and other LAB that may be genetically transformed.

The host cells may be produced by inserting the polynucleotide according to well known techniques, such as is described in the Handbook by Sambrook et al.: Molecular Cloning, A Laboratory Manual (Cold Spring Harbor Laboratory Press, 1989) or Kullen MJ., Klaenhammer TR. Genetic modification of intestinal Lactobacilli and Bifidobacteria. Curr. Issues Mol Biol. 2000;2(2):41-50). The host cells containing at least an endogenous copy of the polynucleotide according to the present invention may be cultivated batch-wise or continuously, e.g. by a batch process (batch cultivation) or by a fed batch process (feed process) or by a repeated fed batch process (repetitive feed process), for the purpose of propagating the genetic material or for synthesizing the polypeptide encoded by the present polynucleotides. A review of known methods of cultivation is given in the textbook by Chmiel (Bioprozesstechnik 1. Einführung in die Bioverfahrenstechnik (Gustav Fischer Verlag, Stuttgart, 1991)) and in the textbook by Storhas (Bioreaktoren und periphere Einrichtungen (Vieweg Verlag, Brunswick/Wiesbaden, 1994)).

The culture medium which is used must fulfil the requirements of the respective micro-organisms used. Descriptions of culture media for various microorganisms are given in the Handbook "Manual of Methods for General Bacteriology" published by the American Society for Bacteriology (Washington D.C., USA, 1981). Suitable sources of carbon include sugar and carbohydrates such as glucose, saccharose, lactose, fructose, maltose, molasses, starch and cellulose, oils and fats such as soya oil, sunflower oil, peanut oil and cocoa fat, fatty acids such as palmitic acid, stearic acid and linoleic acid, alcohols such as glycerol and ethanol, and organic acids such as acetic acid. These substances can be used individually or in admixture. Suitable sources of nitrogen include compounds which contain organic nitrogen, such as peptone, yeast extract, meat extract, malt extract, corn steep liquor, soya bean flour and urea, and inorganic compounds such as ammonium sulphate, ammonium chloride, ammonium phosphate, ammonium carbonate and ammonium nitrate. These sources of nitrogen can be used individually or in admixture. Phosphoric acid, potassium dihydrogen phosphate or dipotassium hydrogen phosphate, or the corresponding sodium-containing salts, can be used as sources of phosphorus. In addition, the culture medium must contain salts of metals such as magnesium sulphate or iron sulphate which are necessary for growth. Finally, essential growth promoting substances such as amino acids and vitamins can be used in addition to the above substances. Moreover, suitable precursors can be added to the culture medium. The aforementioned substances which are used can be added to the culture in the form of a single batch or can be supplied in a suitable manner during cultivation. Basic compounds such as sodium hydroxide, potassium hydroxide, ammonia or aqueous ammonia, or acidic compounds such as phosphoric acid or sulphuric acid are used in a suitable manner in order to control the pH of the culture. Anti-foaming agents such as polyglycol esters of fatty acids can be used to control the generation of foam. In order to maintain the stability of vectors, suitable substances with a selective activity, such as antibiotics, can be added to the medium. In order to maintain aerobic conditions, oxygen or oxygen-containing gas mixtures such as air are passed into the culture. The temperature of the culture normally ranges from 20 °C. to 45 °C. and is preferably 25 ° C. to 40 ° C. Cultivation is continued until a maximum of genetic or protein material has been formed. This target is normally reached within 10 hours to 160 hours.

According to another aspect the present invention also provides polypeptides as identified by SEQ ID. Nos. 4 to 6, or variants thereof, which have a degree of homology to any of the polypeptide of SEQ ID. Nos. 4 to 6 of at least 90 % and are functional, mucin binding polypeptides.

The variants according to invention include polypeptides having the biological activity of binding to mucins and having a homology of at least 90 %, preferably at least 95 %, more preferably at least 98 % to the said polypeptides as identified by SEQ ID. Nos. 4 to 6. These variant polypeptides as used herein shall be understood to mean polypeptides or a fragment thereof which has the above mentioned homology to the sequences shown and which are produced by nucleic acids according to the present invention. These variants may be obtained by deleting, adding or substituting one or more amino acids. Preferably, in case of substituting amino acids, the new amino acids will have properties corresponding or similar to the original ones, particularly with respect to the charge, hydrophobic character, steric properties, etc.

The variant polypeptides of the present invention having mucin binding capacity and homology to the sequences as identified by SEQ ID NOs. 4 to 6 may be produced by conventional mutagenesis or directed evolution procedures. Random mutagenesis of the nucleotide sequence of SEQ ID Nos. 1 to 3 may be effected by several techniques known in the art, such as by chemical mutagenesis, UV or X-ray irradiation, insertion of an oligonucleotide linker randomly into a vector comprising SEQ ID Nos. 1 to 3, or by techniques such as error-prone PCR mutagenesis.

The present invention also comprises chimeric or fusion proteins containing a polypeptide, variants or fragments thereof as described herein. Methods of engineering fragments using recombinant DNA techniques, protein engineering techniques, such as proteolytic digestion, or by protein synthesis are well known in the art. In particular, these fusion proteins may contain, apart from the polypeptide of the present invention, a short sequence of specific amino acids, which will allow a convenient isolation of the polypeptide. Such a tag is e.g. the so-called "His-tag" or another short amino acid sequence having a high antigeneicity and to which antibodies are available having a high binding affinity thereto.

It will be appreciated that the fusion polypeptide according to the present invention may also contain another amino acid sequence having a desired biological activity. Since the polypeptides according to the present invention are surface proteins, i.e. anchored in the micro-organism's cell wall and extending into the environment, the present polypeptides may likewise be utilized for expressing a given polypeptide or peptide having a given biological function in a host cell according to the present invention. To his end, the fusion polypeptide may contain, apart from the amino acid sequence according to he present invention, short peptide sequences, designed for immunizing the host against a given antigen. Likewise, the additional polypeptide part of the present fusion polypeptide may exhibit an enzymatic activity, e.g. a protease activity, which may assist the host in digesting the food material ingested and occasionally also to decrease the allergeneicity of the said food material.

The polynucleotides according to the present invention may be utilized for preparing a polypeptide according to the present invention and also a micro-organism having improved mucin binding capacity.

To this end, a micro-organism of interest is selected and a polynucleotide according to the present invention is inserted therein by recombinant techniques. Preferably, in order to enable a stable propagation of the genetic material introduced, the present construct or polynucleotide is inserted into the hosts chromosome, preferably in a higher copy number. The micro-organism is then subjected to culture conditions allowing expression of the desired polypeptide.

The micro-organisms are preferably micro-organisms, having a beneficial effect on the host incorporating them. Since the inherent task of the gene described herein is to allow a micro-organism to adhere to mucosal tissues, the capability of a micro-organism, expressing such polypeptides, to attach and adhere to the mucosa in a host's organism will be improved. The micro-organism utilized, is preferably a probiotic micro-organism, with the effect that the residence time of the said probiotic micro-organism in the host will be prolonged, while it may exert its beneficial actions for the host for a longer period of time.

Alternatively, in case of a fusion polypeptide having an additional biological function, the micro-organism expressing the present polypeptide will have a novel activity beneficial to the host incorporating them, such as the capacity to display antigens, i.e. its aptitude for being used as a vaccine, or the capacity to degrade proteins, i.e. a protease activity. It will be appreciated that in case of a micro-organism, that is inherently not substantially resistant to conditions in the stomach and therefore not expected to arrive in the gut of an individual in an essentially live and viable form, the skilled person will formulate said micro-organism in a suitable carrier, that enables such a passage.

The present invention also provides a method for the production of a polypeptide according to the present invention which essentially comprises the step of culturing a micro-organism, containing a gene or gene construct as described herein under conditions suitable for growth of the micro-organism and expression of the gene and collecting the polypeptide. This may be achieved by centrifuging the culture broth to recover the micro-organisms as a pellet. The pellet is the subjected to well known treatments in order to isolate the membrane fraction and the proteins contained therein are collected. In order to purify the polypeptide according to the invention antibodies raised against the present polypeptides or antibodies raised against a portion of the fusion protein (e.g. the "tag") may be utilized. Alternatively, in case the polypeptide is synthesized in the micro-organism without its membrane binding domain, the intracellular fraction of the micro-organism is isolated and the proceedings for isolation as exemplarily indicated above are followed. For a more detailed information as regards the isolation of polypeptides from micro-organism reference is made to the Handbook by Sambrook et al.: Molecular Cloning, A Laboratory Manual (Cold Spring Harbor Laboratory Press, 1989)

The present invention also provides a method for detecting a polynucleotide sequence encoding a mucin binding polypeptide and having a polynucleotide sequence homologous to that as identified by SEQ ID. Nos. 1 to 3. Such method comprises the steps of collecting a biological sample, such as e.g. selecting a particular micro-organism. In order to enable good results in the present method, the DNA and/or the RNA of said micro-organism may be isolated prior to performing the detection step. Methods for isolating DNA and/or RNA are described in the Handbook by Sambrook et al.: Molecular Cloning, A Laboratory Manual (Cold Spring Harbor Laboratory Press, 1989). The detection step may be performed by e.g. hybridizing the sample and/or the DNA isolated therefrom with a polynucleotide according to the present invention or a fragment thereof, and detecting the hybridization complex, wherein the presence of the hybridization complex correlates with the presence of a polynucleotide encoding a mucin binding proteins in the biological sample. The detection may be achieved by labeling the polynucleotides used as a probe with e.g. radioactive nucleotides or fluorescence markers and identifying the presence of the label by means of a autoradiography. Alternatively, also the well known technique of the polymerase chain reaction may be utilized. To this end, two oligonucleotides, derived from the present polynucleotide, are chosen such that one oligonucleotide is chosen from the sense and the other from the anti-sense strand such that a fragment, lying between them is defined. The oligonucleotides are then hybridized with the biological sample or the DNA or RNA, respectively isolated therefrom and the fragment lying between them is amplified. The presence of a polynucleotide according to the present invention may be detected by applying an aliquot of the PCR-reaction on a gel and visualizing the fragment obtained, wherein the presence of a fragment, having the appropriate size is indicative of the presence of a mucin binding protein in the biological sample investigated. In order to confirm the results obtained by PCR, the fragment amplified may be sequenced and the nucleic acid sequence thus obtained may be compared with the sequence according to the present invention.

According to an alternative embodiment the invention also provides a method of detecting the presence of a polypeptide according to the present invention in a micro-organism, wherein an antibody raised against the present polypeptides is utilized. The presence of a bound antibody is then indicative of the presence of a mucin binding protein in the micro-organism investigated. It will be appreciated that the skilled person is well aware of raising polyclonal or monoclonal antibodies against a given antigen. In this respect reference is made to Granato D.A., Piguet P.-F. Clin. Exp. Immunol. 1986, 63:703-710, which document is incorporated herein by way of reference.

The following example illustrates the invention without limiting it thereto.

### Example

### Bacterial strains and growth conditions:

*L. johnsonii* CNCM I-1225, *L. johnsonii* 1680, *L. johnsonii* 1657, *L. acidophilus,* CNCM-I-2461, *L. acidophilus* 12, *L. paracasei* CNCM-I-2116, *L. gasseri* 907, *L. reuteri* 2458, *L.* helveticus 1643, were grown under anaerobic conditions in DE Man-Rogosa-Sharpe (MRS) broth (Difco) overnight at 37°C when used for the preparation of antigenic material, ELISA or adhesion assays. All the Lactobacilli were from the Nestlé Culture Collection (Lausanne, Switzerland). Bifidobacteria 362 and CNCM-I 2170 were grown in brain heart infusion (BHI) with cystein (Difco). All the Lactobacilli and Bifidobacteria strains were from the Nestlé Culture Collection (Lausanne, Switzerland).

### Preparation of bacterial surface proteins by guanidinium hydrichloride extraction:

An overnight culture of 40 ml of bacteria was prepared and centrifuged for 10 minutes at 4000 x g at 4°C. The pellet was washed twice with cold phosphate-buffered saline (PBS), and then suspended in 5 M guanidinium hydrochloride (pH7.0) (1 ml/10 mg wet weight). The supernatant fluid was dialyzed overnight against PBS at 4°C in dialysis tubes with a cutoff of 6-8000 Daltons and put at -20°C in small fractions.

### Preparation of bacteria cell wall extract:

An overnight culture of 500 ml of *L. johnsonii* CNCM I-1225 was prepared and centrifuged 10 minutes at 4000 x g at 4°C. The pellet was washed twice with cold phosphate-buffered saline (PBS), and re-suspended in a solution containing 30 mM ammonium carbonate, 1 mM phenylmethylsulfonyl fluoride, 5mM EDTA, 10% sucrose and was adjusted to pH 8. After addition of 2000 U of mutanolysin (Sigma) and 20 mg of lysozyme (109000 U/mg, Sigma), the suspension was incubated for 30-60 min at 37 °C. Monitoring of protoplasts formation was done by reading the decrease of optical density measured at 590 nm (OD decreases until protoplast formation is finished). The suspension was then centrifuged at 10 000g for 10 minutes at 4°C. The supernatant was dialyzed against 50 mM ammonium bicarbonate pH 7.0, divided in small fractions and kept at - 20°C.

### Cell culture:

Caco-2 human intestinal cells (American Type Culture Collection) were used between passages 40 and 90. Cells were routinely grown in Dulbecco's modified Eagle minimal essential medium (25 mM glucose) (Gibco BRL) supplemented with 20% heat-inactivated (30, 56°C) fetal calf serum (Gibco, BRL) and 1% nonessential amino acids (Gibco, BRL) as previously described (7, 8, 12, 15-17). Cells were cultured at 37°C in a 10% CO₂-90% air atmosphere. The culture medium was changed every 2 days. Cells were used at postconfluence after 15 days of culturing (fully differentiated cells) (44).

The colonic adenocarcinoma cell line HT29 was obtained from American type Culture Collection (ATCC, manassas, VA). Undifferentiated cells were maintained in glucose-containing DMEM supplemented with 10% fetal calf serum (FCS; Animed Bioconcept, Allschwill, Switzerland) at 37 °C in a 5% CO₂- air atmosphere. Culture medium was changed every 2 days.

### Preparation of anti CNCM I- 1225 surface antibodies:

Rabbit serum against live CNCM I-1225 (10⁹ /injection) was prepared by EUROGENTEC (B-4040 Herstal-Belgium). The IgG fraction of 20 ml of antiserum from the final bleeding was precipitated with a final concentration of 33% of ammonium sulfate and dialysed against PBS. The IgG were then incubated for 2 hours at 4 °C with a washed suspension of an overnight culture of 200 ml of CNCM I-1225 in PBS. After three washing steps with PBS, the bound antibodies were eluted with 0.1 M glycine-HCl pH 2.3 and neutralized immediately with 1M Tris-HCl pH 8. The antibody solution was then applied onto a FPLC Protein A Sepharose column (Pharmacia, Amersham) and eluted according the conditions of the manufacturer. Briefly, 4 ml aliquots were applied at 1ml/min in 0.1 M Tris-HCl buffer pH 8. After washing in the same buffer, IgG were eluted with 0.1 M glycine-HCl buffer pH 3. The 1 ml fractions were immediately neutralized with 0.2 ml of 1 M Tris-HCl buffer pH 8. The peak was dialyzed against PBS and kept in small fractions at - 20 °C.

### Detection of rabbit anti CNCM I-1225 antibodies:

L. johnsonii bacteria from an overnight culture were centrifuged, washed 2 times with PBS pH 7.2 and adjusted to an optical density of 10 at 650 (OD₆₅₀) with PBS. For coating, 0.3 ml of this suspension was diluted to a final volume of 10 ml with a 0.05 M carbonate-bicarbonate buffer (pH 9.6) (coating buffer). Maxisorb polyvinyl wells (Nunc) were coated overnight at 4 °C with 100 µl of this suspension. After saturation with gelatin (10 mg/ml I PBS), bacteria on the wells were incubated for 2 hours at room temperature on a rotating shaker with 100 µl dilutions of rabbit dilutions of anti live CNCM I-1225 or anti total CNCM I-1225 Ig. After three washings with PBS pH 7.2 containing 0.05% Tween 20, 100 µl of goat anti-rabbit Ig conjugated to alkaline phosphatase (Sigma) diluted 1/2000 in PBS-Tween 20 were added to the wells for 1 hr at room temperature. After three more washings in the same buffer, the substrate p-nitro-phenyl phosphate (Sigma) dissolved in the appropriate buffer was added to the wells. Readings of enzymatic activity were made at 405 nm after 2 hr at 37 °C with a Dynatech MR 5000 microtiter plate reader.

### Mucin purification from HT29-MT/:

Purification was done according to Lesuffleur at al. (Lesuffleur, T., Barbat, A., Dussaulx, E., Zweibaum, A. 1990. Growth adaptation to methotrexate of HT-29 human colon carcinoma cells is associated with their ability to differentiate into columnar absorptive and mucus-secreting cells. Cancer Res. 50: 6334).

In this respect, HT29-MTX (1) were cultured in 10 cm diameter plates for 3 weeks in DMEM cell medium (Gibco) supplemented with 10% fetal calf serum (Gibco). Cell culture medium was replaced by serum-free medium for 16 to 24 h. Cell culture supernatants and mucus were collected by repeated gentle pipetting on cells without damaging the monolayer. Samples were centrifuged 10 min at 1'200 rpm to discard dead cells, and supernatants were further centrifuged 30 min at 15'000 rpm to discard cellular debris. To avoid protein degradation, 0.01% NaN₃, 5 mM EDTA, and 2 mM PMSF were added to the supernatants. Samples were stored at -20°C until use or were concentrated by lyophilization and further resuspended in 0.4 ml of 100 mM ammonium bicarbonate pH 7.6/HT29-MTX plate. They were loaded onto a Sepharose CL-4B column (Pharmacia) equilibrated in the same buffer (10 ml of Sepharose gel/ HT29-MTX plate). Elution of the column was done at a flow rate of 0.5 ml/min. Mucin containing fractions were eluted in the void volume, pooled and lyophilized.

### Mucin quantification and analysis:

Total protein concentration was determined using the BCA kit (Pierce) or the Bio-Rad protein assay (Bio-Rad) according to manufacturers' instructions. Total sugars were assessed as described (Dubois M, Gilles KA, Hamilton JK, Rebers, PA, Smith, F. Colorimetric method for determination of sugars and related substances. *Anal Chem* 68: 350-6, 1956. Mucin preparations were analyzed by SDS/PAGE. For protein analysis, 10-20 µg of purified mucins were loaded onto a 5% SDS/PAGE gel and revealed by silver staining. For sugar analysis, 10-20 µg purified mucins were loaded onto a 4-20% gradient SDS/PAGE gel and revealed by PAS (Periodic acid Schiff) staining.

### Detection of CNCM I-1225 adhesion proteins:

T75 flasks containing differentiated Caco-2 cells or Ht29 cells were washed 2 times either with PBS pH 7.2 or 0.05 M sodium acetate buffer pH 5 containing 0.1M NaCl. They were then incubated with 12 ml PBS or acetate buffer alone or containing 100 µg/ml of CNCM I-1225 cell wall extract for 1 h at room temperature. After three washes with the respective buffers, they were harvested with a rubber policeman and transferred first in 50 ml tubes, washed once more and put into 1.5 ml Eppendorf tubes. To elute the CNCM I-1225 adhesins, the pellets were gently resuspended in 1 ml of 0.1 M glycine HCl pH 3 and centrifuged 5 minutes at 5000 rpm in a Sorvall Biofuge 13 centrifuge. The supernatants were immediately neutralized with 0.2 ml of 1 M Tris-HCl buffer pH 8 and dialyzed against PBS diluted three times overnight. They were then concentrated to 300 µl. The samples (20 µl) and the CNCM I-1225 cell wall extract were then applied onto a 4-20 % SDS polyacrylamide gel (Bio-Rad) in four series in non reducing conditions and blotted onto PVDF membranes. One part was stained with Coomassie Blue. The rest of the membrane was saturated with 3% BSA in 0.1 M Tris-HCL buffer pH 7.2, containing 0.15 M NaCl and 0.1 % Tween-20 (Buffer I) 2 h at 37 °C. The sample series were then incubated with 2 µg/ml of anti CNCM I-1225 specific surface antibodies, anti CNCM I-1225 lysate IgG antibodies or specific anti-ovalbumin antibodies (control) overnight at 4 °C. After three 15 minutes washings, goat anti rabbit Ig (whole molecule) conjugated to alkaline phosphatase (Sigma) was added for one hr at room temperature. After three more washings, CNCM I-1225 proteins that were bound to the cells were revealed by the BCIP/NBT substrate Kit (Zymed Laboratories, San Francisco, CA) according to the conditions of the manufacturer.

### Protein sequencing:

The CNCM I-1225 protein band corresponding to a protein of apparent molecular weight of 58 kDa (P58) detected with the anti CNCM I-1225 surface antibodies was excised from the PVDF membrane stained with Coomassie blue and the first twelve amino acids were sequenced. The result gave Ala-Glu-Lys-Gln-Val-Tyr-Glu-Arg-Thr-Lys-Ala-Leu. This P58 peptide sequence was compared to the total predicted translated proteins of CNCM I-1225 using the bioinformatic program BlastP, which returned a strong match to the gene CNCM I-1225-009. The translated protein of CNCM I-1225-009 was compared to the current protein databases using the program BlastP and which returned a significant 77% identity (expectation value 0.0) to the elongation factor Tu (efTu) of *Bacillus stearothermophilus* [Ref. Krasny L., Mesters J.R., Tieleman L.N., Kraal B., Fucik V., RA Hilgenfeld R., Jonak J. "Structure and expression of elongation factor Tu from Bacillus stearothermophilus." J. Mol. Biol. 283:371-381(1998).], involved in translation of proteins. This result is unexpected, as efTu is expected to be present only within the cell, and not on the cell surface.

The CNCM I-1225 protein band corresponding to a protein of apparent molecular weight of 80 kDa (P80) detected with the anti CNCM I-1225 lysate antibodies was excised from the PVDF membrane stained with Coomassie blue and the first twelve amino acids were sequenced according to Edman. The result gave Met-Lys-Lys-Thr-Lys-Ile-Val-Ser-Thr-Leu-Gly-Pro. This P80 peptide sequence was compared to the total predicted translated proteins of CNCM I-1225 using the program BlastP, which returned a perfect match to the amino-terminus of the gene CNCM I-1225-080. The translated protein was compared to the current protein databases using the program BlastP and which returned a significant 81 % identity (expectation value 0.0) to the *Lactobacillus delbrueckii* subsp. bulgaricus pyruvate kinase (Accession number P34038) (Branny P, De La Torre F, Garel JR. (1996) The genes for phosphofructokinase and pyruvate kinase of *Lactobacillus delbrueckii* subsp. bulgaricus constitute an operon. J. Bacteriol 178:4727-30) involved in carbon metabolism. This result is unexpected, as pyruvate kinase is expected to be present only within the cell, and not on the cell surface.

The CNCM I-1225 protein band corresponding to a protein of apparent molecular weight of 90 kDa (P90) detected with the anti CNCM I-1225 lysate antibodies was excised from the PVDF membrane stained with Coomassie blue and the first twelve amino acids were sequenced according to Edman. The result gave Asn(?)-Tyr-Phe-Asp-Asn-Gly-Asn-Tyr-Asn-Phe-Asp-Asp. This P90 peptide sequence was compared to the total predicted translated proteins of CNCM I-1225 using the program BlastP, which returned a perfect match to the amino-terminus of the gene CNCM I-1225-775. The translated protein was compared to the current protein databases using the program BlastP and which returned a significant 47% identity (expectation value e-162) to the *Lactococcus lactis* clp protease subunit E protein [Bolotin, A. et. al. (2001) The complete genome sequence of the lactic acid bacterium Lactococcus lactis ssp. lactis IL1403. Genome Res. 11 (5), 731-753] involved in degradation of incorrectly folded proteins. This result is unexpected, as clpE is expected to be present only within the cell, and not on the cell surface.

### Expression Cloning:

To order to elucidate the biological activity of the efTu gene or the gene product, respectively, the efTu was expressed in the heterologous host E. coli. The efTu gene was modified to remove the secretion signal sequence at the amino terminus of the produced protein to reduce toxic effects of overexpression, while the translation termination codon was eliminated and replaced by a peptide linker plus six times histidine tag to aid purification and detection of the recombinant protein. To achieve this objective, efTu gene was PCR amplified from L. johnsonii CNCM I-1225 chromosomal DNA using the oligonucleotide primers D363 (5'ATATATTCATGACAGAAAAGAACATTACG3'), introducing a BspHI restriction site (underlined), and D364 (5'ATATATGGATCCTCAAGGATTTCAGTAACTTGACC3') introducing a BamHI restriction site (underlined).

To achieve the cloning and overexpression of the pyruvate kinase, the pyruvate kinase gene was PCR amplified from *L. johnsonii* CNCM I-1225 chromosomal DNA using the oligonucleotide primers D123 (5'ATATATCATGAAGAAAACTAAAATTGTTAGTAC3'), introducing a BspHI restriction site (underlined), and D81 (5'AATTCAGGATCCAGGTTAGTTGCTTCGCC3') introducing a BamHI restriction site (underlined).

To achieve the cloning and overexpression of the protease subunit clpE, the clpE gene was PCR amplified from *L. johnsonii* CNCM I-1225 chromosomal DNA using the oligonucleotide primers D124 (5'ATATATCCATGGCATACTTTGACAATGGAA3'), introducing a BspHI restriction site (underlined), and D83 (5'ATTGCTGGATCCTTTTCTTCTTTTTTATCAGC3') introducing a BamHI restriction site (underlined).

PCR was performed using the Pwo thermostable polymerase (Roche Molecular Biochemicals) on an Applied Biosystems 9700 thermocycler using the following amplification protocol: incubate at 95°C for 5 min, followed by 30 cycles of 95°C for 30 sec, 50°C for 30 sec, 68°C for 3 min and finally incubated at 68°C for 7 min before holding at 4°C. The amplicon was purified using the Millipore spin column system and the fragment eluted in 50 µl of TE buffer (10 mM Tris pH 8.0, 1 mM EDTA). A 25 µl sample was digested to completion with the restriction enzymes BspHI plus BamHI (or NcoI plus BamHI for clpE), the desired fragment purified over a 1% agarose gel, extracted from the agarose using the Life Technologies Concert gel extraction system and eluted in a 50 µl volume of TE. This fragment was ligated into the E. coli expression vector pET24d (Novagen) previously digested with the restriction enzymes Ncol plus *Bam*HI and dephosphorylated. The ligation was transformed into the E. coli strain XL1 blue (Stratagene) and transformants were selected on LB plates supplemented with Kanamycin. Individual colonies were screened by small-scale plasmid preparation and restriction enzyme analysis. Plasmids showing the expected restriction pattern were subjected to DNA sequence analysis to confirm the integrity of the construction. One DNA sequence confirmed plasmid was designated pDP649 as shown below.

Plasmid pDP649 was transformed into the expression host BL21 (DE3) codon plus RIL obtained from Stratagene Inc and transformants selected on LB agar plates supplemented with 50 µg/ml Kanamycin and 30 µg/ml chloramphenicol. The host BL21 (DE3) codon plus RIL contains a plasmid carrying the chloramphenicol resistance selectable marker plus the arginine (AGA), isoleucine (ATT) and leucine (TTA) tRNA genes to promote the over expression of *L. johnsonii* CNCM I-1225 genes which is relatively A/T rich and hence containing these potentially problematic anticodons.

### Production of the CNCM I-1225 proteins:

Luria Bertani broth culture of 500 ml were grown overnight at 37°C to an optical density (abs. 600 nm) of 0.6 OD and protein expression was induced by the addition of 1 mM IPTG. Four hours after induction, the bacteria were harvested by centrifugation at 4000 x g for 20 min at 4 °C and resuspended in 5 ml of lysis buffer (50 mM NaH₂PO₄, 300 mM NaCl, 5 mM Imidazole pH 8) of pellet, containing 1 mg/ml of egg lysozyme (Fluka). After an incubation of 30 min on ice, the suspension was sonicated on ice 6 x 15 " with 15 " intervals at 50% of amplitude. The lysate was centrifuged at 10 000 x g for 30 min to pellet cellular debris. The His-Tagged protein contained in the supernatant was purified in native conditions by passage over a nickel affinity resin (Ni-NTA, Qiagen). The column was equilibrated in lysis buffer and charged with the bacterial lysate. After washing with lysis buffer containing 10 mM imidazole until there was no more absorbance at 280 nm, the protein was eluted in 500 µl fractions with 125 mM imidazole in the same buffer. Monitoring of the elution was done with the Bio-Rad protein assay. The pooled protein fractions were equilibrated in PBS by passage onto a PD10 column (Pharmacia, Amersham), and frozen at -20 °C in presence of 10% glycerol until further use.

### Preparation of polyclonal antibodies against the CNCM I-1225 recombinant protein:

Balb/c mice aged 6-8 weeks were injected subcutaneously with 20 µg of recombinant protein absorbed on alum 2 times at weekly intervals and then with the same amount of protein in PBS at 15 days interval. Serum samples from mice were tested by ELISA or western blot analysis for reactivity against the antigen. Normal mouse serum was obtained from non-immunized mice from the same lot.

### Binding of the CNCM I-1225 recombinant efTu, clpE and pyruvate kinase to HT29 or Caco-2 cells at pH 5 or 7.2:

HT29 (10000 cells/well) or Caco-2 cells (20 000 cells/well) were cultivated in 96- well cell culture NUNCLON Surface flat bottom plates (Nunc) in their respective medium for one or two weeks. They were then washed 2 times with PBS or 0.05M sodium acetate, 0.1 M NaCl buffer pH 5.0. CNCM I-1225 cell wall extract or recombinant proteins (100 µl) diluted in the respective buffers containing 10 mg/BSA were added to the cells and incubated 2 h at room temperature. Following three washings, cells were fixed with 200 µl of 2.5% paraformaldehyde in PBS for 10 min at room temperature. After three washings, cells were incubated with 100 µl of 1 µg/ml of mouse anti HisTag antibody (Penta.His Antibody, Qiagen, Basel) or mouse antisera anti CNCM I-1225 recombinant proteins diluted 1/2000, followed by rabbit Ig anti mouse Ig conjugated to peroxydase (Zymed) 1/2000 in PBS for 1 hour at room temperature. After three more washings, the enzymatic activity bound to the wells was revealed with a TMB (tetramethyl benzidine) substrate kit (Pierce) and measured at 450 nm.

### Binding of the CNCM I-1225 recombinant efTu, clpE and pyruvate kinase to HT29 MTX mucins at pH 5 or 7.2:

Maxisorb 96 polyvinyl wells (Nunc) were coated with 50 µl of 5 µg/ml of HT29 MTX mucin preparation in 0.05 M carbonate/bicarbonate buffer pH 9.6 overnight at 4 °C.

After saturation with 200 µl of 10 mg/ml of gelatine in PBS, the plates were washed two times with PBS pH 7.2 or 0.05M sodium acetate, 0.1 M NaCl buffer pH 5. CNCM I-1225 cell wall extract or recombinant proteins (100 µl) diluted in the respective buffers containing 10 mg/ BSA were added to the wells and incubated 2 h at 37 °C. Following three washings, wells were fixed with 200 µl of 2.5% paraformaldehyde in PBS for 10 min at room temperature. After three washings, 100 µl of 1 µg/ml of mouse anti HisTag antibody ( Penta-His Antibody, Qiagen, Basel) or mouse antisera anti CNCM I-1225 recombinant proteins diluted 1/2000 in PBS were added to the wells and incubated for 1 h at 37 °C, followed by rabbit Ig anti mouse Ig conjugated to peroxydase (Zymed )1/2000 in PBS for 1 hour at 37°C. After three more washings, the enzymatic activity bound to the wells was revealed with a TMB (tetramethyl benzidine) substrate kit (Pierce) and measured at 450 nm.

### Detection of efTu in outer membrane proteins or S-layer of Lactobacilli and Bidobacteria:

Samples (1 µg) preparations from *L. johnsonii* CNCM I-1225, Lj3, La16, *L. acidophilus,* La10, La27, *L. paracasei* ST11, *L. gasseri* LGA4, *L. reuteri* 2458, *L. helveticus* LH157, and from Bifidobacteria B129 and Bb12, were run onto 10% SDS acrylamide gels in reducing conditions and blotted on 0.2 µm nitrocellulose (Biorad). After saturation as described above, the blots were incubated overnight at 4 °C with anti pyruvate kinase, anti clpE and anti efTu mouse antisera at dilutions of 1/10000, 1/5000 and 1/5000 respectively and normal mouse serum diluted 1/5000 as control. An additional blot was incubated with a monoclonal antibody obtained by injection of a guanidine-HCl extract of CNCM I-1225 into Balb/c mice. After washings, the blots were incubated for 1 hour at room temperature with rabbit anti mouse Ig conjugated to alkaline phosphatase (Santa Cruz Biotechnology, Inc) diluted 1/2000. Detection of the enzymatic activity to the blots was done with the BCIP/NBT substrate Kit (Zymed Laboratories, San Francisco, CA) according to the conditions of the manufacturer.

### Measure of the isoelectric point of efTu in CNCM I-1225 cell wall extract:

Cell wall extracts (2 µg) and recombinant efTu (1 µg) were diluted with distilled water to 100 µl and applied on Ampholine PAG plate, pH 3-10 (Amersham, Pharmacia, Biotech, Inc). After migration as described by the manufacturer, they were blotted on 0.2 µm nitrocellulose with a Transblot SD Semi-Dry Electrophoretic Transfer Cell (Biorad) Detection of the efTu protein was done as described above.

### Detection and identification of CNCM I-1225 cell wall proteins binding to differentiated Caco-2 cells and HT29 cells:

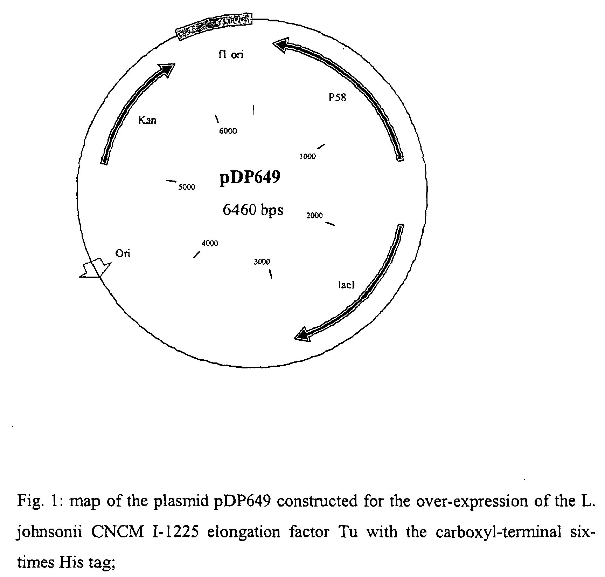

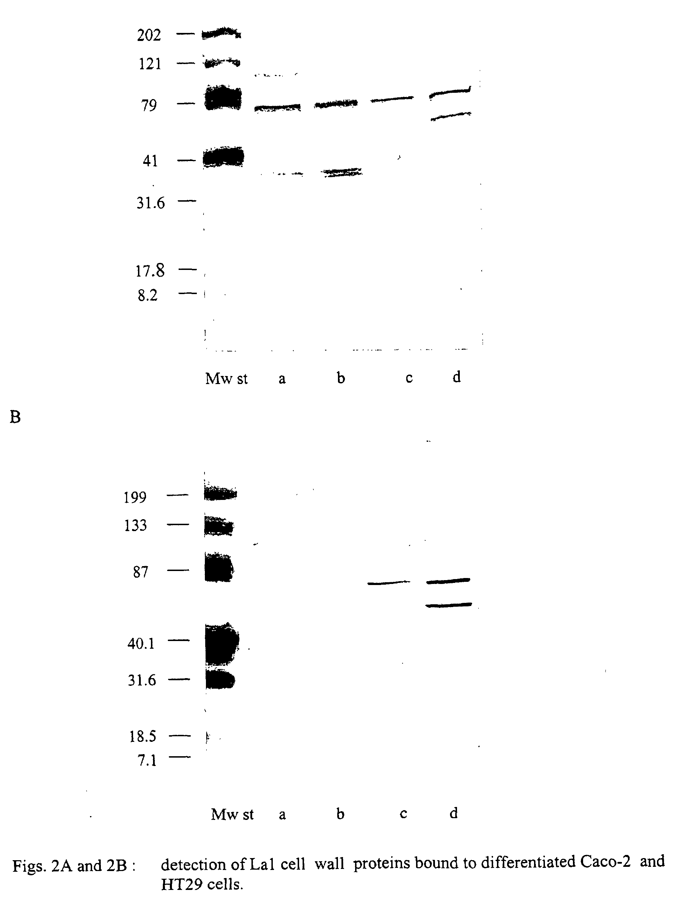

After the incubation with Caco-2 cells at different pHs, CNCM I-1225 proteins bound to their surface were eluted from the cells and resolved on a 4-20% SDS acrylamide gels together with the control cells eluates in non reducing conditions. They were blotted onto PVDF membranes and detected with anti-CNCM I-1225 surface antibodies. One of them of apparent molecular weight of 60 kD was found in the eluate from Caco-2 cells incubated at pH 5.0 only with CNCM I-1225 cell wall extract (Fig. 1 A). A protein band with the same apparent molecular weight was detected with the same antibodies in the eluate of HT29 cells incubated at pH 5.0 with the same cell wall extract (Fig. 1B). In order to confirm these results, the specific eluates from Caco-2 and HT29 cells incubated with the CNCM I-1225 cell wall extract at pH 5 were run onto SDS 4-20% acrylamide gels, blotted and incubated with the antisera produced against the recombinant of this protein. As shown on fig 2, (has to be done again) the presence of this CNCM I-1225 protein could be detected on the immunoblots from Caco-2 or HT29 cells eluates.

### Binding of efTu recombinant protein to Caco-2 and HT29 cells:

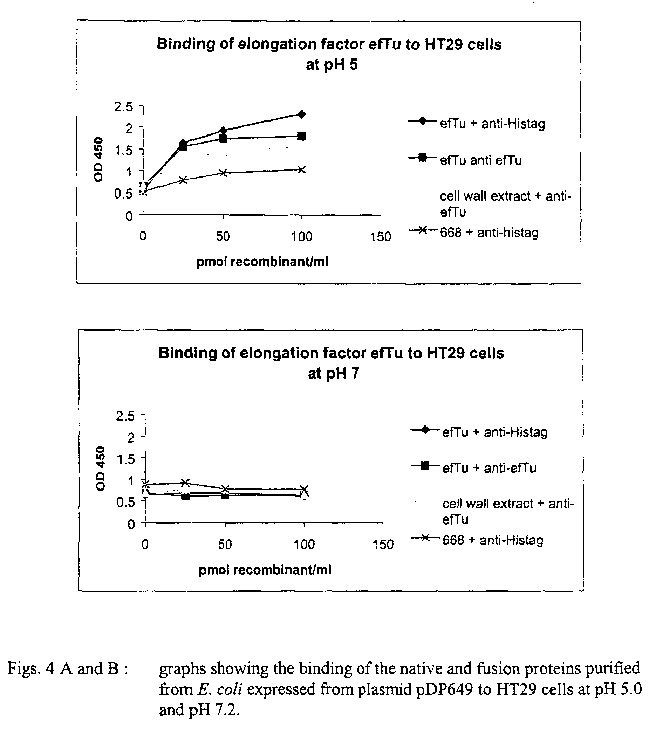

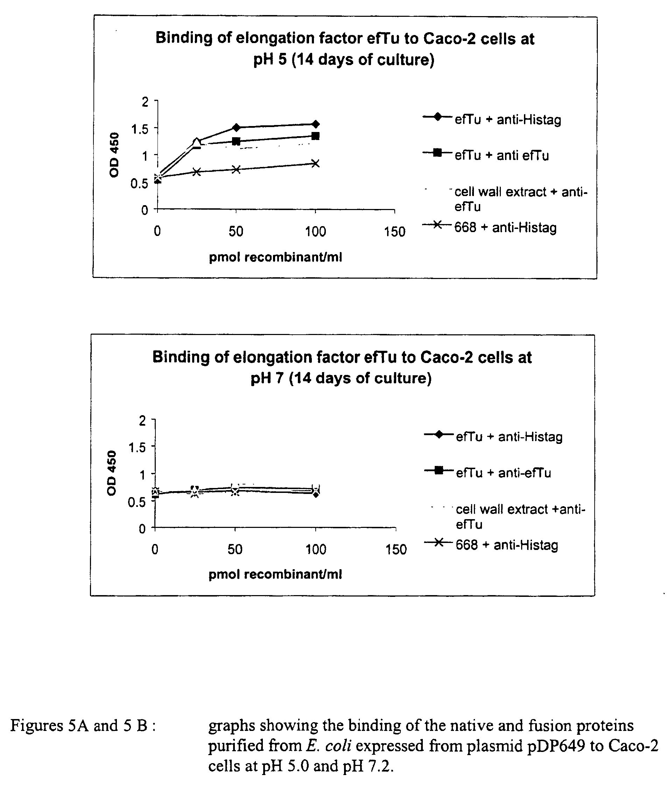

Binding of the efTu recombinant was done at 2 pH on both cell lines. Detection of the bound molecule was done by an antibody directed against the "His-Tag" epitope of the recombinant protein or by a polyclonal antibody directed against the whole molecule. As can be seen in Figs. 4 and 5, the efTu recombinant molecule did bind to both cell lines at pH 5, whereas it did not bind at pH 7.2. To confirm that the "His-Tag" was not responsible for the binding, a cell wall extract of CNCM I-1225 lactobacilli was incubated at different dilutions with the cells. The presence of efTu bound to the cells at pH 5 was again detected by the polyclonal antibody, excluding thus a non specific binding due to the "His-Tag".

### Binding of efTu recombinant protein to HT29 MTX mucins:

The same binding experiment was done with the efTu recombinant or the CNCM I-1225 cell wall extract and a purified preparation of human intestinal cells mucins. As seen in Fig 5, the binding pattern of efTu to mucins was similar to the one observed in Fig 4 and 5 with the cell lines. The finding that the efTu molecule binds to its receptor only at acidic pH would suggest an electrostatic interaction between them. As by isoelectrofocusing, the isoelectric point of the recombinant molecule and the native efTu were found to be 5.6 and 5.8 respectively, their binding occurred when they were positively charged and not when they were negatively charged at pH 7.2. Only the isolation of efTu receptor on Caco-2 or HT29 cells will allow to determine whether another kind of binding can also take place.

### Detection of efTu in CNCM I-1225 and other bacteria cell surface associated proteins:

In order to elucidate whether efTu was covalently bound to peptidoglycan or associated to the surface of CNCM I-1225 bacteria in a non-covalent way, these bacteria and a set of other bacteria for comparison were treated with guanidine-HCl as described above. The released proteins were run on SDS acrylamide gels, blotted on nitrocellulose and incubated with the anti efTu antibody. It could be shown that this protein may be found in surface associated proteins of most lactobacilli and to a lesser extent in two Bifidobacteria.

SEQUENCE LISTING

<110> Société des Produits Nestlé S. A.

<120> Novel mucin binding polypeptides derived from Lactobacillus johnsonii

<130> 80409/EP

<140> EP 02019243.1
<141> 2002-08-27

<160> 15

<170> PatentIn version 3.1

<210> 1
<211> 1191
<212> DNA
<213> Lactobacillus johnsonii

<400> 1
atggcagaaa aagaacatta cgaaagaact aagccgcacg ttaacattgg tactatcggc 60

cacgttgacc atggtaagac cactttaact gcagctatta ctactgtttt ggcagaagac 120

ggtttggcac aagctgaaga ttactcacaa atcgatgctg ctccagaaga aaaggaacgt 180

ggtattacta ttaataccgc tcacgtagaa tacgaaacta agaatcgtca ctacgctcac 240

atggatgctc caggtcacgc tgactacatc aagaacatga ttactggtgc tgcacaaatg 300

gatggtgcta tcttggttgt tgctgcaact gatggtccta tgccacaaac tcgtgaacac 360

atcttacttg ctcgtcaagt tggtgttcaa tacatcgttg tattcttaaa caaggttgac 420

ttagttgacg atccagaatt gatcgactta gttgaaatgg aagtacgtga cttgttatca 480

gaatacgatt accctggtga cgatgttcct gttatccgtg gttcagcttt gaaggcactt 540

gaaggtgatc cagaacaaca agacgttatc agaaaattaa tggaaactgt tgacgaatac 600

attccaactc cagaacgtga tactgacaag ccattcttaa tgccagttga agacgtattt 660

actatcactg gtcgtggtac tgttgcttct ggtcgtatcg accgtggtac tgttaaggtc 720

ggcgatgaag ttgaaatcgt tggtttaact gacaagattg aaaagtcaac tgttactggt 780

ttggaaatgt tccacaagac tcttgacctt ggtgaagccg gcgataacgt tggtgtatta 840

cttcgtggta tcgaccgtga tcaagttgaa cgtggtcaag ttttagctgc acctggttca 900

attcaaaccc acaagaactt caaggtcaa gtttatatct tgaacaaaga tgaaggtggt 960

cgtcacactc cattcttctc agattaccgt ccacaattct acttccacac tactgacgta 1020

actggtaaga ttgaattgcc agaaggtact gaaatggtta tgcctggtga taacgttgaa 1080

ttcactgttg aattgatcaa gccagttgcc attgaaaagg gtactaagtt tactatccgt 1140

gaaggtggta agactgttgg tgctggtcaa gttactgaaa tccttgacta a 1191

<210> 2

<211>  1770
<212>  DNA
<213>  Lactobacillus johnsonii

<400>  2
atgaagaaaa ctaaaattgt tagtacttta gggccagcct caaacgatgt tgagactatt      60

actaaattag ctgaagcagg tgcaaacgta ttccgtttca acttctcaca cggtgaccac     120

gaagaacacc gtgcacgtat gaacatggtt cgtgaggttg aaaagaagac tggtaaactt     180

cttggcattg ctcttgatac taagggtgcc gaaattagaa ctactgaaca agaaggcggc     240

aagttcacta ttaacactgg tgacactatc cgtatttcta tggacgacac taagaagggt     300

aacaaggaca tgatccacgt tacctacgat ggtctttacg acgatactca cgttggtggt     360

cacgtattaa tcgatgatgg tttagttgac cttttgatta aggacaaaga tgacgctaag     420

agagaattag tttgtgaagc tcaaaacact ggtgtaatcg gttctaagaa gggtgttaac     480

gctcctggcg ttgaaatccg cctcccaggt attactgaaa aagatactaa cgatattaag     540

ttcggtttga aggaaggtat caacttcatc tttgcttcat tcgttcgtaa agcacaagat     600

gttcttgaca ttcgtcaatt gcttgaagaa gcacattgtg aatacgtaaa gatcttccct     660

aagattgaat cacaagaagg tattgacaac atcgacgaaa tcttaaaggt ttctgatggt     720

ttaatggttg ctcgtggtga catgggtgtt gaaattccat ttatcaatgt tccatttgta     780

caaaaagaat tgatcaagag atgtaacgct ttaggtaagc cagttattac tgctactcaa     840

atgcttgact caatgcaaga aaacccacgt cctactcgtg ccgaagttac tgacgttgcc     900

aacgctgttc ttgatggtac tgacgcaact atgctttcag gtgaatctgc aaacggtctt     960

tacccagtta aagctgttaa ggctatggct gaaatcgaca tgcgtactga aaagcaactt    1020

gctaagcgta acaagttagc tcttcaaaga ttcgaagaat acaagggttc aaacgttact    1080

gaagctatcg gtgaatcagt tgttagaact gctgaagaat tgggcgttaa gactattatt    1140

gctgcaactg aatctggtta cactgcacgt atgatttcta agtaccgtcc aaacgcagac    1200

attattgcta tgactttcaa cgacaagatt caacactcat taggtgttgt ttggggtgta    1260

aagccaatgt tgactgaaaa gcctaagtct actgaagaaa tgtttgaaaa ggcagctgaa    1320

cttgctaaag aaactggtct tgcaaaagac ggtgacttag taattatcgt tgctggtgtt    1380

cctttgggcg aaactggtac tactaacttg atgaagttac aattaatcgg tacccaactt    1440

gttaaaggtt taggtgttgg tgaacaatca gttattggta aagctgtagt tgcaacttct    1500

gctgaagaag ctaacagcaa agttcaagat ggtgacatct tagttgctaa gactactgat    1560

aaggattact taccagcaat caagaaggct tcaggtatgg tagttgaagc ttctggttta    1620

actagccatg cagctgttgt tggtctttca cttggtatcc cagttatcgt tggtgctact    1680

gatgcagtag aaaagatcaa ggatggttct aagattactg ttgatgctcg tcgcggtgct    1740

gtttaccaag gcgaagcaac taacctttaa 1770

<210> 3
<211> 2115
<212> DNA
<213> Lactobacillus johnsonii

<400> 3
atggcatact ttgacaatgg aaactataat tttgatgatt tgtttaatga attaaacaga 60

gattttttca atgatggaag aatgactagt ggtcaacaac caatgagata tagtgctggt 120

caagcacctc aacaaggcgc tcaaaatggc caacaaaagg aaaagccaat tggtgttgat 180

ttaactgaac aagctaagaa gggtaaatat gatcctgtca ttggacgtac cgctgttatt 240

gatcaagtta ttgaaatttt gagtagacgt aaaaagaaca atcctgtttt gactggtcct 300

gctggtgttg gtaaaacctc ggtagttgaa ggattagctc aaagaattgt tgatggagac 360

gttcctgaaa aactaaagaa tgctcatatt attgagttaa atattaatga attagttgca 420

ggtacttctc ttcgtggtag ttttgaagaa aaactaaaga agattatcga taaggctaag 480

ggtgacaaga atgttattct ctttatcgat gaattacata atattgttgg tgcaggttct 540

actgattcag aaaataattc tggggatgct gctaatatct taaagccagc attagctagc 600

ggtgaaattc gtgttattgg tgcaactact actagtgaat accaacaaat cgaaaaagac 660

cctgctttag ctagaagatt ccaaccagtt caagttccag aaccaactat caaggatgct 720

attcaaattt tgaagggatt agctccaaga tatgaaaaat accacaatgt taaatatgat 780

ccagaagctt tgaagcttgc tgcagaactt tcaaatcgtt acattaatga tcgtcactta 840

ccagataagg ccattgactt aatggacgaa gctggtgcta agaagtcctt aggtatggat 900

caaaaagatg aaacttcaat taagaacaag attcatgcct tagaaggtaa aaaggcagaa 960

gcagctaaga aggaaaaata tgatgaagct gctaagatta agaaagagat tgaagacctt 1020

aagaaagatc ttaagaatgc cggcaaagct agtgaactta aagttacacc agaagatatg 1080

tatgctttaa ttcaaactaa gactggtatt ccaatgagtg aaattaatgc aaatgaagct 1140

caaaagaatg ctaacttagc ttctgacttg aagaaagttg ttattgacca agacaaggcc 1200

attgacatca ttactgatgc aattgcacgt aagcaagtat ttaaggattc taaccgtcca 1260

actggttcat tcttacttac tggtcctact ggtgttggta agactgaatt agctaagcaa 1320

ttagcaatta agttatttgg taaggcagat gcattggtac gtttggatat gtcagaatat 1380

caagaccaaa tggctgttaa taagttgatc ggttcagcac ctggttatgt tggctatggc 1440

gaaggtggtc aattaactga aaagattcgt caccaaccat attcattgat tttgcttgat 1500

gaaattgaaa aagcaaatcc tcaagtcttc aatgctttgc ttcaaatcat ggatgatggt 1560

cgtttaactg atgcacaagg tagaacaatt tccttcaaag atacaatcat tatcatgact 1620

tcaaatgctg gatactctga caagttactt aagggtgacg atcaagatgc tttacgtaaa 1680

gctcttgaag tttacttcag accagaattc ttaaaccgtc ttgacgcaat tgttccattt 1740

aattctttaa ctagtgaaga tatgctcaag atcttagacc tttacttagt aaagatggag 1800

aagtctcttg ctaagagaaa cattaagctt catgttactg atgatgctaa gaagtacttg 1860

gctaagaagg gttatgacaa ggaatttggt gctcgtccat tgagaagagt tgttgaacaa 1920

gatcttgaaa ccccagcagc taagttgatt gtttctaagc cagatactaa ggaagttaaa 1980

ttcactattg atgataagca tttgtactta aatggtgaaa ttcttgaaga tgtattgttt 2040

gacaaggaaa ctcgtgaaca agaaaagaaa gacatcaagg aagctgaaaa agctgataaa 2100

aaagaagaaa aatag 2115

<210> 4
<211> 396
<212> PRT
<213> Lactobacillus johnsonii

<400> 4

Met Ala Glu Lys Glu His Tyr Glu Arg Thr Lys Pro His Val Asn Ile
1               5                   10                  15

Gly Thr Ile Gly His Val Asp His Gly Lys Thr Thr Leu Thr Ala Ala
            20                  25                  30

Ile Thr Thr Val Leu Ala Glu Asp Gly Leu Ala Gln Ala Glu Asp Tyr
        35                  40                  45

Ser Gln Ile Asp Ala Ala Pro Glu Glu Lys Glu Arg Gly Ile Thr Ile
    50                  55                  60

Asn Thr Ala His Val Glu Tyr Glu Thr Lys Asn Arg His Tyr Ala His
65                  70                  75                  80

Met Asp Ala Pro Gly His Ala Asp Tyr Ile Lys Asn Met Ile Thr Gly
                85                  90                  95

Ala Ala Gln Met Asp Gly Ala Ile Leu Val Val Ala Ala Thr Asp Gly
            100                 105                 110

Pro Met Pro Gln Thr Arg Glu His Ile Leu Leu Ala Arg Gln Val Gly
        115                 120                 125

Val Gln Tyr Ile Val Val Phe Leu Asn Lys Val Asp Leu Val Asp Asp
    130                 135                 140

Pro Glu Leu Ile Asp Leu Val Glu Met Glu Val Arg Asp Leu Leu Ser

145                 150                 155                 160

Glu Tyr Asp Tyr Pro Gly Asp Asp Val Pro Val Ile Arg Gly Ser Ala
                165                 170                 175

Leu Lys Ala Leu Glu Gly Asp Pro Glu Gln Gln Asp Val Ile Arg Lys
            180                 185                 190

Leu Met Glu Thr Val Asp Glu Tyr Ile Pro Thr Pro Glu Arg Asp Thr
        195                 200                 205

Asp Lys Pro Phe Leu Met Pro Val Glu Asp Val Phe Thr Ile Thr Gly
    210                 215                 220

Arg Gly Thr Val Ala Ser Gly Arg Ile Asp Arg Gly Thr Val Lys Val
225                 230                 235                 240

Gly Asp Glu Val Glu Ile Val Gly Leu Thr Asp Lys Ile Glu Lys Ser
                245                 250                 255

Thr Val Thr Gly Leu Glu Met Phe His Lys Thr Leu Asp Leu Gly Glu
            260                 265                 270

Ala Gly Asp Asn Val Gly Val Leu Leu Arg Gly Ile Asp Arg Asp Gln
        275                 280                 285

Val Glu Arg Gly Gln Val Leu Ala Ala Pro Gly Ser Ile Gln Thr His
    290                 295                 300

Lys Asn Phe Lys Gly Gln Val Tyr Ile Leu Asn Lys Asp Glu Gly Gly
305                 310                 315                 320

Arg His Thr Pro Phe Phe Ser Asp Tyr Arg Pro Gln Phe Tyr Phe His
                325                 330                 335

Thr Thr Asp Val Thr Gly Lys Ile Glu Leu Pro Glu Gly Thr Glu Met
            340                 345                 350

Val Met Pro Gly Asp Asn Val Glu Phe Thr Val Glu Leu Ile Lys Pro
        355                 360                 365

Val Ala Ile Glu Lys Gly Thr Lys Phe Thr Ile Arg Glu Gly Gly Lys
    370                 375                 380

Thr Val Gly Ala Gly Gln Val Thr Glu Ile Leu Asp
385                 390                 395

<210> 5
<211> 589
<212> PRT
<213> Lactobacillus johnsonii

<400> 5

Met Lys Lys Thr Lys Ile Val Ser Thr Leu Gly Pro Ala Ser Asn Asp
1               5                   10                  15

Val Glu Thr Ile Thr Lys Leu Ala Glu Ala Gly Ala Asn Val Phe Arg
            20                  25                  30

Phe Asn Phe Ser His Gly Asp His Glu Glu His Arg Ala Arg Met Asn
        35                  40                  45

Met Val Arg Glu Val Glu Lys Lys Thr Gly Lys Leu Leu Gly Ile Ala
    50                  55                  60

Leu Asp Thr Lys Gly Ala Glu Ile Arg Thr Thr Glu Gln Glu Gly Gly
65                  70                  75                  80

Lys Phe Thr Ile Asn Thr Gly Asp Thr Ile Arg Ile Ser Met Asp Asp
                85                  90                  95

Thr Lys Lys Gly Asn Lys Asp Met Ile His Val Thr Tyr Asp Gly Leu
            100                 105                 110

Tyr Asp Asp Thr His Val Gly Gly His Val Leu Ile Asp Asp Gly Leu
        115                 120                 125

Val Asp Leu Leu Ile Lys Asp Lys Asp Asp Ala Lys Arg Glu Leu Val
    130                 135                 140

Cys Glu Ala Gln Asn Thr Gly Val Ile Gly Ser Lys Lys Gly Val Asn
145                 150                 155                 160

Ala Pro Gly Val Glu Ile Arg Leu Pro Gly Ile Thr Glu Lys Asp Thr
                165                 170                 175

Asn Asp Ile Lys Phe Gly Leu Lys Glu Gly Ile Asn Phe Ile Phe Ala
            180                 185                 190

Ser Phe Val Arg Lys Ala Gln Asp Val Leu Asp Ile Arg Gln Leu Leu
        195                 200                 205

Glu Glu Ala His Cys Glu Tyr Val Lys Ile Phe Pro Lys Ile Glu Ser
    210                 215                 220

Gln Glu Gly Ile Asp Asn Ile Asp Glu Ile Leu Lys Val Ser Asp Gly
225                 230                 235                 240

Leu Met Val Ala Arg Gly Asp Met Gly Val Glu Ile Pro Phe Ile Asn
                245                 250                 255

Val Pro Phe Val Gln Lys Glu Leu Ile Lys Arg Cys Asn Ala Leu Gly
            260                 265                 270

Lys Pro Val Ile Thr Ala Thr Gln Met Leu Asp Ser Met Gln Glu Asn
        275                 280                 285

Pro Arg Pro Thr Arg Ala Glu Val Thr Asp Val Ala Asn Ala Val Leu
    290                 295                 300

Asp Gly Thr Asp Ala Thr Met Leu Ser Gly Glu Ser Ala Asn Gly Leu
305                 310                 315                 320

Tyr Pro Val Lys Ala Val Lys Ala Met Ala Glu Ile Asp Met Arg Thr
                325                 330                 335

Glu Lys Gln Leu Ala Lys Arg Asn Lys Leu Ala Leu Gln Arg Phe Glu
            340                 345                 350

Glu Tyr Lys Gly Ser Asn Val Thr Glu Ala Ile Gly Glu Ser Val Val
        355                 360                 365

Arg Thr Ala Glu Glu Leu Gly Val Lys Thr Ile Ile Ala Ala Thr Glu
    370                 375                 380

Ser Gly Tyr Thr Ala Arg Met Ile Ser Lys Tyr Arg Pro Asn Ala Asp
385                 390                 395                 400

Ile Ile Ala Met Thr Phe Asn Asp Lys Ile Gln His Ser Leu Gly Val
                405                 410                 415

Val Trp Gly Val Lys Pro Met Leu Thr Glu Lys Pro Lys Ser Thr Glu
            420                 425                 430

Glu Met Phe Glu Lys Ala Ala Glu Leu Ala Lys Glu Thr Gly Leu Ala
        435                 440                 445

Lys Asp Gly Asp Leu Val Ile Ile Val Ala Gly Val Pro Leu Gly Glu
    450                 455                 460

Thr Gly Thr Thr Asn Leu Met Lys Leu Gln Leu Ile Gly Thr Gln Leu
465                 470                 475                 480

Val Lys Gly Leu Gly Val Gly Glu Gln Ser Val Ile Gly Lys Ala Val
                485                 490                 495

Val Ala Thr Ser Ala Glu Glu Ala Asn Ser Lys Val Gln Asp Gly Asp
            500                 505                 510

Ile Leu Val Ala Lys Thr Thr Asp Lys Asp Tyr Leu Pro Ala Ile Lys
        515                 520                 525

Lys Ala Ser Gly Met Val Val Glu Ala Ser Gly Leu Thr Ser His Ala
    530                 535                 540

Ala Val Val Gly Leu Ser Leu Gly Ile Pro Val Ile Val Gly Ala Thr
545                 550                 555                 560

Asp Ala Val Glu Lys Ile Lys Asp Gly Ser Lys Ile Thr Val Asp Ala
                565                 570                 575

Arg Arg Gly Ala Val Tyr Gln Gly Glu Ala Thr Asn Leu
            580                 585

<210>  6
<211>  692
<212>  PRT
<213>  Lactobacillus johnsonii

<400>  6

Met Ala Tyr Phe Asp Asn Gly Asn Tyr Asn Phe Asp Asp Leu Phe Asn
1               5                   10                  15

Glu Leu Asn Arg Asp Phe Phe Asn Asp Gly Arg Met Thr Ser Gly Gln
            20                  25                  30

Gln Pro Met Arg Tyr Ser Ala Gly Gln Ala Pro Gln Gly Ala Gln Asn
        35                  40                  45

Gly Gln Gln Lys Glu Lys Pro Ile Gly Val Asp Leu Thr Glu Gln Ala
    50                  55                  60

Lys Lys Gly Lys Tyr Asp Pro Val Ile Gly Arg Thr Ala Val Ile Asp
65                  70                  75                  80

Gln Val Ile Glu Ile Leu Ser Arg Arg Lys Lys Asn Asn Pro Val Leu
                85                  90                  95

Thr Gly Pro Ala Gly Val Lys Thr Ser Val Val Glu Gly Leu Ala Gln
            100                 105                 110

Arg Ile Val Asp Gly Asp Val Pro Glu Lys Leu Lys Asn Ala His Ile
        115                 120                 125

Ile Glu Leu Asn Ile Asn Glu Leu Val Ala Gly Thr Ser Leu Arg Gly
    130                 135                 140

Ser Phe Glu Glu Lys Leu Lys Lys Ile Ile Asp Lys Ala Lys Gly Asp
145                 150                 155                 160

Asn Val Ile Leu Phe Ile Asp Glu Leu His Asn Ile Val Gly Ala Gly
                165                 170                 175

Ser Thr Asp Ser Glu Asn Asn Ser Gly Asp Ala Ala Asn Ile Leu Lys
            180                 185                 190

Pro Ala Leu Ala Ser Gly Glu Ile Arg Val Ile Gly Ala Thr Thr Thr
        195                 200                 205

Ser Glu Tyr Gln Gln Ile Glu Lys Asp Pro Leu Ala Arg Arg Phe Gln
    210                 215                 220

Pro Val Gln Val Pro Glu Pro Thr Ile Lys Asp Ala Ile Gln Ile Leu
225                 230                 235                 240

Lys Gly Leu Ala Pro Arg Tyr Glu Lys Tyr His Asn Val Lys Tyr Asp
                245                 250                 255

Pro Glu Ala Leu Lys Leu Ala Ala Glu Leu Ser Asn Arg Tyr Ile Asn
            260                 265                 270

Asp Arg His Leu Asp Lys Ala Ile Asp Leu Met Asp Glu Ala Gly Ala
        275                 280                 285

Lys Lys Ser Leu Gly Met Asp Gln Lys Asp Glu Thr Ser Ile Lys Asn
    290                 295                 300

Lys Ile His Ala Leu Glu Gly Lys Lys Ala Glu Ala Ala Lys Lys Glu
305                 310                 315                 320

Lys Tyr Asp Glu Ala Ala Lys Ile Lys Lys Glu Ile Glu Asp Lys Lys
                325                 330                 335

Asp Leu Lys Asn Ala Gly Lys Ala Ser Glu Leu Lys Val Thr Pro Glu
            340                 345                 350

Asp Met Tyr Ala Leu Ile Gln Thr Lys Thr Gly Ile Pro Met Ser Glu
        355                 360                 365

Ile Asn Ala Asn Glu Ala Gln Lys Asn Ala Asn Leu Ala Ser Asp Leu
    370                 375                 380

Lys Lys Val Val Ile Asp Gln Asp Ala Ile Asp Ile Ile Thr Asp Ala
385                 390                 395                 400

Ile Ala Arg Lys Gln Val Phe Lys Asp Ser Asn Arg Pro Thr Gly Ser
                405                 410                 415

Phe Leu Leu Thr Gly Pro Thr Gly Val Gly Lys Thr Glu Leu Ala Lys
            420                 425                 430

Gln Leu Ala Ile Lys Leu Phe Gly Lys Ala Asp Ala Leu Val Arg Leu
        435                 440                 445

Asp Met Glu Tyr Gln Asp Gln Met Ala Val Asn Lys Leu Ile Gly Ser
    450                 455                 460

Ala Pro Gly Tyr Val Gly Tyr Gly Glu Gly Gly Gln Leu Thr Glu Lys
465                 470                 475                 480

Ile Arg His Gln Pro Tyr Ser Leu Ile Leu Leu Asp Glu Ile Glu Lys
                485                 490                 495

Ala Asn Pro Gln Val Phe Asn Ala Leu Leu Gln Ile Asp Asp Gly Arg
            500                 505                 510

Leu Thr Asp Ala Gln Gly Arg Thr Ile Ser Phe Lys Asp Thr Ile Ile
        515                 520                 525

Ile Met Thr Ser Asn Ala Gly Tyr Ser Asp Lys Leu Leu Lys Gly Asp
    530                 535                 540

Asp Gln Asp Ala Leu Arg Lys Ala Leu Glu Val Tyr Phe Arg Pro Glu
545                 550                 555                 560

Phe Leu Asn Arg Leu Asp Ile Val Pro Phe Asn Ser Leu Thr Ser Glu
                565                 570                 575

Asp Met Leu Lys Ile Leu Asp Leu Tyr Leu Val Lys Met Glu Lys Ser
            580                 585                 590

Leu Ala Lys Arg Asn Ile Lys Leu His Val Thr Asp Asp Ala Lys Lys
        595                 600                 605

Tyr Leu Ala Lys Lys Gly Tyr Asp Lys Glu Phe Gly Ala Arg Pro Leu

610                 615                 620

Arg Val Val Glu Gln Asp Leu Glu Thr Pro Ala Ala Lys Leu Ile Val
625                 630                 635                 640

Ser Lys Pro Asp Thr Lys Glu Val Lys Phe Thr Ile Asp Asp Lys His
                645                 650                 655

Leu Tyr Leu Asn Gly Glu Ile Leu Glu Asp Val Leu Phe Asp Lys Glu
            660                 665                 670

Thr Arg Glu Gln Glu Lys Lys Asp Ile Lys Ala Glu Lys Ala Asp Lys
        675                 680                 685

Lys Glu Glu Lys
    690

<210>  7
<211>  12
<212>  PRT
<213>  Lactobacillus johnsonii

<400>  7

Ala Glu Lys Gln Val Tyr Glu Arg Thr Lys Ala Leu
1               5                   10

<210>  8
<211>  12
<212>  PRT
<213>  Lactobacillus johnsonii

<400>  8

Met Lys Lys Thr Lys Ile Val Ser Thr Leu Gly Pro
1               5                   10

<210>  9
<211>  12
<212>  PRT
<213>  Lactobacillus johnsonii

<220>
<221>  misc_feature
<222>  (1)..(1)
<223>  Xaa = Asn (?)

<400>  9

Xaa Tyr Phe Asp Asn Gly Asn Tyr Asn Phe Asp Asp
1               5                   10

<210>  10

<211>  30
<212>  DNA
<213>  Artificial Sequence

<220>
<223>  oligonucleotide primer D363

<400>  10
atatattcat gacagaaaaa gaacattacg                                        30


<210>  11
<211>  35
<212>  DNA
<213>  Artificial Sequence

<220>
<223>  oligonucleotide primer D364

<400>  11
atatatggat cctcaaggat ttcagtaact tgacc                                  35


<210>  12
<211>  33
<212>  DNA
<213>  Artificial Sequence

<220>
<223>  oligonucleotide primer D123

<400>  12
atatatcatg aagaaaacta aaattgttag tac                                    33


<210>  13
<211>  29
<212>  DNA
<213>  Artificial Sequence

<220>
<223>  oligonucleotide primer D81

<400>  13
aattcaggat ccaggttagt tgcttcgcc                                         29


<210>  14
<211>  30
<212>  DNA
<213>  Artificial Sequence

<220>
<223>  oligonucleotide primer  D124

<400>  14
atatatccat ggcatacttt gacaatggaa                                        30


<210>  15
<211>  32
<212>  DNA
<213>  Artificial Sequence

<220>
<223> oligonucleotide primer D83

<400> 15
attgctggat ccttttcttc ttttttatca gc 32

SEQID 1: La1 efTu gene sequence

ATGGCAGAAAAAGAACATTACGAAAGAACTAAGCCGCACGTTAACATTGGTACTATCGGC
CACGTTGACCATGGTAAGACCACTTTAACTGCAGCTATTACTACTGTTTTGGCAGAAGAC
GGTTTGGCACAAGCTGAAGATTACTCACAAATCGATGCTGCTCCAGAAGAAAAGGAACGT
GGTATTACTATTAATACCGCTCACGTAGAATACGAAACTAAGAATCGTCACTACGCTCAC
ATGGATGCTCCAGGTCACGCTGACTACATCAAGAACATGATTACTGGTGCTGCACAAATG
GATGGTGCTATCTTGGTTGTTGCTGCAACTGATGGTCCTATGCCACAAACTCGTGAACAC
ATCTTACTTGCTCGTCAAGTTGGTGTTCAATACATCGTTGTATTCTTAAACAAGGTTGAC
TTAGTTGACGATCCAGAATTGATCGACTTAGTTGAAATGGAAGTACGTGACTTGTTATCA
GAATACGATTACCCTGGTGACGATGTTCCTGTTATCCGTGGTTCAGCTTTGAAGGCACTT
GAAGGTGATCCAGAACAACAAGACGTTATCAGAAAATTAATGGAAACTGTTGACGAATAC
ATTCCAACTCCAGAACGTGATACTGACAAGCCATTCTTAATGCCAGTTGAAGACGTATTT
ACTATCACTGGTCGTGGTACTGTTGCTTCTGGTCGTATCGACCGTGGTACTGTTAAGGTC
GGCGATGAAGTTGAAATCGTTGGTTTAACTGACAAGATTGAAAAGTCAACTGTTACTGGT
TTGGAAATGTTCCACAAGACTCTTGACCTTGGTGAAGCCGGCGATAACGTTGGTGTATTA
CTTCGTGGTATCGACCGTGATCAAGTTGAACGTGGTCAAGTTTTAGCTGCACCTGGTTCA
ATTCAAACCCACAAGAACTTCAAGGGTCAAGTTTATATCTTGAACAAAGATGAAGGTGGT
CGTCACACTCCATTCTTCTCAGATTACCGTCCACAATTCTACTTCCACACTACTGACGTA
ACTGGTAAGATTGAATTGCCAGAAGGTACTGAAATGGTTATGCCTGGTGATAACGTTGAA
TTCACTGTTGAATTGATCAAGCCAGTTGCCATTGAAAAGGGTACTAAGTTTACTATCCGT
GAAGGTGGTAAGACTGTTGGTGCTGGTCAAGTTACTGAAATCCTTGACTAA

SEQID 2: La1 pyruvate kinase gene sequence

ATGAAGAAAACTAAAATTGTTAGTACTTTAGGGCCAGCCTCAAACGATGTTGAGACTATT
ACTAAATTAGCTGAAGCAGGTGCAAACGTATTCCGTTTCAACTTCTCACACGGTGACCAC
GAAGAACACCGTGCACGTATGAACATGGTTCGTGAGGTTGAAAAGAAGACTGGTAAACTT
CTTGGCATTGCTCTTGATACTAAGGGTGCCGAAATTAGAACTACTGAACAAGAAGGCGGC
AAGTTCACTATTAACACTGGTGACACTATCCGTATTTCTATGGACGACACTAAGAAGGGT
AACAAGGACATGATCCACGTTACCTACGATGGTCTTTACGACGATACTCACGTTGGTGGT
CACGTATTAATCGATGATGGTTTAGTTGACCTTTTGATTAAGGACAAAGATGACGCTAAG
AGAGAATTAGTTTGTGAAGCTCAAAACACTGGTGTAATCGGTTCTAAGAAGGGTGTTAAC

GGTGAAATTCGTGTTATTGGTGCAACTACTACTAGTGAATACCAACAAATCGAAAAAGAC
CCTGCTTTAGCTAGAAGATTCCAACCAGTTCAAGTTCCAGAACCAACTATCAAGGATGCT
ATTCAAATTTTGAAGGGATTAGCTCCAAGATATGAAAAATACCACAATGTTAAATATGAT
CCAGAAGCTTTGAAGCTTGCTGCAGAACTTTCAAATCGTTACATTAATGATCGTCACTTA
CCAGATAAGGCCATTGACTTAATGGACGAAGCTGGTGCTAAGAAGTCCTTAGGTATGGAT
CAAAAAGATGAAACTTCAATTAAGAACAAGATTCATGCCTTAGAAGGTAAAAAGGCAGAA
GCAGCTAAGAAGGAAAAATATGATGAAGCTGCTAAGATTAAGAAAGAGATTGAAGACCTT
AAGAAAGATCTTAAGAATGCCGGCAAAGCTAGTGAACTTAAAGTTACACCAGAAGATATG
TATGCTTTAATTCAAACTAAGACTGGTATTCCAATGAGTGAAATTAATGCAAATGAAGCT
CAAAAGAATGCTAACTTAGCTTCTGACTTGAAGAAAGTTGTTATTGACCAAGACAAGGCC
ATTGACATCATTACTGATGCAATTGCACGTAAGCAAGTATTTAAGGATTCTAACCGTCCA
ACTGGTTCATTCTTACTTACTGGTCCTACTGGTGTTGGTAAGACTGAATTAGCTAAGCAA
TTAGCAATTAAGTTATTTGGTAAGGCAGATGCATTGGTACGTTTGGATATGTCAGAATAT
CAAGACCAAATGGCTGTTAATAAGTTGATCGGTTCAGCACCTGGTTATGTTGGCTATGGC
GAAGGTGGTCAATTAACTGAAAAGATTCGTCACCAACCATATTCATTGATTTTGCTTGAT
GAAATTGAAAAAGCAAATCCTCAAGTCTTCAATGCTTTGCTTCAAATCATGGATGATGGT
CGTTTAACTGATGCACAAGGTAGAACAATTTCCTTCAAAGATACAATCATTATCATGACT
TCAAATGCTGGATACTCTGACAAGTTACTTAAGGGTGACGATCAAGATGCTTTACGTAAA
GCTCTTGAAGTTTACTTCAGACCAGAATTCTTAAACCGTCTTGACGCAATTGTTCCATTT
AATTCTTTAACTAGTGAAGATATGCTCAAGATCTTAGACCTTTACTTAGTAAAGATGGAG
AAGTCTCTTGCTAAGAGAAACATTAAGCTTCATGTTACTGATGATGCTAAGAAGTACTTG
GCTAAGAAGGGTTATGACAAGGAATTTGGTGCTCGTCCATTGAGAAGAGTTGTTGAACAA
GATCTTGAAACCCCAGCAGCTAAGTTGATTGTTTCTAAGCCAGATACTAAGGAAGTTAAA
TTCACTATTGATGATAAGCATTTGTACTTAAATGGTGAAATTCTTGAAGATGTATTGTTT
GACAAGGAAACTCGTGAACAAGAAAAGAAAGACATCAAGGAAGCTGAAAAAGCTGATAAA
AAAGAAGAAAAATAG

SEQID 4: La1 efTu polypeptide sequence

MAEKEHYERTKPHVNIGTIGHVDHGKTTLTAAITTVLAEDGLAQAEDYSQIDAAPEEKER
GITINTAHVEYETKNRHYAHMDAPGHADYIKNMITGAAQMDGAILVVAATDGPMPQTREH
ILLARQVGVQYIVVFLNKVDLVDDPELIDLVEMEVRDLLSEYDYPGDDVPVIRGSALKAL
EGDPEQQDVIRKLMETVDEYIPTPERDTDKPFLMPVEDVFTITGRGTVASGRIDRGTVKV
GDEVEIVGLTDKIEKSTVTGLEMFHKTLDLGEAGDNVGVLLRGIDRDQVERGQVLAAPGS
IQTHKNFKGQVYILNKDEGGRHTPFFSDYRPQFYFHTTDVTGKIELPEGTEMVMPGDNVE

FTVELIKPVAIEKGTKFTIREGGKTVGAGQVTEILD

SEQID 5: La1 pyruvate kinase polypeptide sequence

MKKTKIVSTLGPASNDVETITKLAEAGANVFRFNFSHGDHEEHRARMNMVREVEKKTGKL
LGIALDTKGAEIRTTEQEGGKFTINTGDTIRISMDDTKKGNKDMIHVTYDGLYDDTHVGG
HVLIDDGLVDLLIKDKDDAKRELVCEAQNTGVIGSKKGVNAPGVEIRLPGITEKDTNDIK
FGLKEGINFIFASFVRKAQDVLDIRQLLEEAHCEYVKIFPKIESQEGIDNIDEILKVSDG
LMVARGDMGVEIPFINVPFVQKELIKRCNALGKPVITATQMLDSMQENPRPTRAEVTDVA
NAVLDGTDATMLSGESANGLYPVKAVKAMAEIDMRTEKQLAKRNKLALQRFEEYKGSNVT
EAIGESVVRTAEELGVKTIIAATESGYTARMISKYRPNADIIAMTFNDKIQHSLGVVWGV
KPMLTEKPKSTEEMFEKAAELAKETGLAKDGDLVIIVAGVPLGETGTTNLMKLQLIGTQL
VKGLGVGEQSVIGKAVVATSAEEANSKVQDGDILVAKTTDKDYLPAIKKASGMVVEASGL
TSHAAVVGLSLGIPVIVGATDAVEKIKDGSKITVDARRGAVYQGEATNL

SEQID 6: La1 clpE polypeptide sequence

MAYFDNGNYNFDDLFNELNRDFFNDGRMTSGQQPMRYSAGQAPQGAQNGQQKEKPIGVDL
TEQAKKGKYDPVIGRTAVIDQVIEILSRRKKNNPVLTGPAGVKTSVVEGLAQRIVDGDVP
EKLKNAHIIELNINELVAGTSLRGSFEEKLKKIIDKAKGDNVILFIDELHNIVGAGSTDS
ENNSGDAANILKPALASGEIRVIGATTTSEYQQIEKDPLARRFQPVQVPEPTIKDAIQIL
KGLAPRYEKYHNVKYDPEALKLAAELSNRYINDRHLDKAIDLMDEAGAKKSLGMDQKDET
SIKNKIHALEGKKAEAAKKEKYDEAAKIKKEIEDKKDLKNAGKASELKVTPEDMYALIQT
KTGIPMSEINANEAQKNANLASDLKKVVIDQDAIDIITDAIARKQVFKDSNRPTGSFLLT
GPTGVGKTELAKQLAIKLFGKADALVRLDMEYQDQMAVNKLIGSAPGYVGYGEGGQLTEK
IRHQPYSLILLDEIEKANPQVFNALLQIDDGRLTDAQGRTISFKDTIIIMTSNAGYSDKL
LKGDDQDALRKALEVYFRPEFLNRLDIVPFNSLTSEDMLKILDLYLVKMEKSLAKRNIKL
HVTDDAKKYLAKKGYDKEFGARPLRVVEQDLETPAAKLIVSKPDTKEVKFTIDDKHLYLN
GEILEDVLFDKETREQEKKDIKAEKADKKEEK

## Claims

1. A polynucleotide as identified by any of SEQ. ID. Nos. 1 to 3 or variants or fragments thereof, obtainable by deleting, adding or substituting one or more nucleotides, having a degree of homology to SEQ. ID. No. 1 of at least 90 % and encoding a functional mucin binding polypeptide.

2. The polynucleotide according to claim 1, which has a degree of homology to SEQ. ID. No. 1 to 3 of at least 95 %, preferably 98 %.

3. The polynucleotide according to claim 1 or 2 , which has a sequence as identified by SEQ ID. Nos. 1, 2 or 3.

4. A vector comprising a polynucleotide sequence according to any of the preceding claims.

5. The vector according to claim 4, which is an expression vector.

6. A host cell harboring a recombinant polynucleotide according to any of the claims 1 to 3 or a vector according to any of the claims 4 or 5.

7. The host cell according to claim 6, which is a procaryotic or an eucaryotic cell.

8. A polypeptide as identified by any of SEQ ID. Nos. 4 to 6, or variants thereof, obtained by deleting, adding or substituting one or more amino acids, which have a degree of homology to the polypeptide of any of SEQ ID. Nos. 4 to 6 of at least 90 % and are a functional, mucin binding polypeptide.

9. The polypeptide according to claim 8, which have a degree of homology to the polypeptide as identified by any of SEQ. ID. Nos. 4 to 6 of at least 95 % preferably 98 %.

10. The polypeptide according to claim 8, which has a sequence as identified by SEQ. ID. No. 4, 5 or 6.

11. A fusion-polypeptide containing a polypeptide according to any of the claims 8 to 10.

12. Use of a polynucleotide according to any of the claims 1 to 3 or a vector according to any of the claims 4 or 5 for the preparation of a polypeptide.

13. Use of a polynucleotide according to any of the claims 1 to 3 or a vector according to any of the claims 4 or 5 for the preparation of a micro-organism exhibiting improved mucin binding capacity.

14. The use according to claim 12, wherein the micro-organism is a probiotic microorganism.

15. A method for producing a polypeptide according to any of the claims 8 to 11, which comprises, culturing a host cell according to any of the claims 7 or 8 under conditions, which allow synthesis of the polypeptide and recovering the polypeptide.

16. An antibody, capable to bind to a polypeptide according to any of the claims 8 to 11.

17. A method for detecting a mucin binding protein in a biological sample, which comprises the steps
(a) providing a biological sample;
(a) optionally purifying the DNA contained in the biological sample; and
(b) detecting the presence of a polynucleotide according to any of the claims 1 to 3.

18. A method for detecting a mucin binding protein in a biological sample, which comprises the steps
(b) providing a biological sample;
(c) detecting the presence of a mucin binding polypeptide according to any of the claims 8 to 11.
